(19) Europäisches Patentamt
European Patent Office
Office européen des brevets

(11) EP 1 798 218 A1

(12) **EUROPEAN PATENT APPLICATION**
published in accordance with Art. 158(3) EPC

(43) Date of publication:
**20.06.2007 Bulletin 2007/25**

(21) Application number: **05788232.6**

(22) Date of filing: **29.09.2005**

(51) Int Cl.:
***C07C 235/80*** *(2006.01)* ***C07C 231/12*** *(2006.01)*
***C08F 20/36*** *(2006.01)* ***C09D 133/14*** *(2006.01)*

(86) International application number:
**PCT/JP2005/018043**

(87) International publication number:
**WO 2006/035915 (06.04.2006 Gazette 2006/14)**

(84) Designated Contracting States:
**DE GB**

(30) Priority: **29.09.2004 JP 2004284402**
**21.12.2004 JP 2004368920**

(71) Applicant: **KANSAI PAINT CO., LTD.**
**Amagasaki-shi, Hyogo-ken 661-8555 (JP)**

(72) Inventor: **KOBATA, Masami,**
**Kansai Paint Co., Ltd.**
**Kanagawa, 2548562 (JP)**

(74) Representative: **Kuhnen & Wacker**
**Intellectual Property Law Firm**
**Prinz-Ludwig-Strasse 40A**
**85354 Freising (DE)**

(54) **MONOMER COMPOUND, PROCESS FOR PRODUCING THE SAME, POLYMER THEREOF, AND WATER-BASED CURABLE COMPOSITION**

(57) The present invention provides an α-oxoamide group-and (meth)acryloyloxy group-containing monomer compound represented by Formula (I)

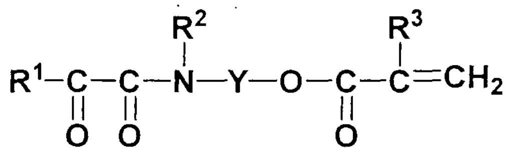

(I)

wherein Y is a $C_{1-6}$ straight-chain alkylene group which may be substituted with a $C_{1-6}$ organic group, $R^1$ is a $C_{1-16}$ organic group, $R^2$ is a hydrogen atom or a $C_{1-8}$ organic group, and $R^3$ is a hydrogen atom or a methyl group; a production process therefor; a polymer thereof; and an aqueous curable composition containing the polymer.

EP 1 798 218 A1

**Description**

TECHNICAL FIELD

The present invention relates to a carbonyl-containing monomer compound, a process for producing the same, a polymer of the compound, and an aqueous curable composition containing the polymer.

BACKGROUND ART

Compounds having carbonyl groups together with (meth)acryloyloxy groups or (meth)acrylamide groups are easily polymerizable, and are useful as monomers for introducing carbonyl groups to polymers.

Known such carbonyl-containing monomers include, for example, N-(1,1-dimethyl-3-oxobutyl)acrylamide (conventionally known as diacetone acrylamide), acetoacetoxylethyl methacrylate (also known as 2-(methacryloyloxy)ethylacetoacetate), etc.

N-(1,1-Dimethyl-3-oxobutyl)acrylamide is usually produced by reacting acrylonitrile, concentrated sulfuric acid, and acetone, as described, for example, in Japanese Unexamined Patent Application Publication No. 2000-159736.

However, the above production process has problems in that acrylonitrile, which is highly toxic and highly volatile, is likely to pollute the air around the production facilities; and in that since highly oxidative and highly corrosive concentrated sulfuric acid is used in a large amount, the production operation involves danger. Carbonyl-containing monomers that can be produced without using acrylonitrile or concentrated sulfuric acid as a starting material are therefore desired.

Acetoacetoxylethyl methacrylate can be produced from t-butyl acetoacetate and 2-hydroxyethyl methacrylate without requiring acrylonitrile or concentrated sulfuric acid as a starting material. Thus, the production operation for acetoacetoxylethyl methacrylate is less dangerous than the that for N-(1,1-dimethyl-3-oxobutyl)acrylamide.

Prog. Org. Coat., 27, 73-78 (1996) discloses that a curable composition obtained by adding a crosslinking agent that is reactive with carbonyl groups to a carbonyl-containing polymer prepared by using acetoacetoxylethyl methacrylate as a monomer is useful as a room temperature curable aqueous composition.

However, as described in, for example, J. Coat. Technol., 70 (881), 57 (1998), polymers and copolymers of acetoacetoxylethyl methacrylate have the problem of poor storage stability since, in water, they are decomposed with time by a reverse Claisen condensation reaction of acetoacetoxy groups or like reaction. The reason why acetoacetoxy groups undergo a reverse reaction of Claisen condensation is presumably that two carbonyl groups in acetoacetoxy groups have β-diketo-type structure and one of the carbonyl groups is an ester carbonyl group. Therefore, carbonyl-containing monomers that do not have β-diketo-type structure and do not undergo a reverse Claisen condensation reaction are desired.

DISCLOSURE OF THE INVENTION

PROBLEMS TO BE SOLVED BY THE INVENTION

An object of the present invention is to provide a carbonyl-containing monomer compound which can be safely produced using low toxic starting materials and whose polymers have excellent storage stability.

Another object of the present invention is to provide a production process for the above compound.

A further object of the present invention is to provide a polymer of the above compound.

A still further object of the present invention is to provide an aqueous curable composition comprising the above polymer and a specific crosslinking compound.

Other objects and features of the present invention will become apparent from the following description.

MEANS FOR SOLVING THE PROBLEMS

The present inventors conducted extensive research to achieve the above objects. As a result, they found that a specific monomer compound containing an α-oxoamide group and a (meth)acryloyloxy group achieves all of the above objects, and accomplished the present invention.

The present invention provides the following monomer compounds, production processes therefor, polymers thereof, and aqueous curable compositions containing the polymers.

1. A monomer compound containing an α-oxoamide group and a (meth)acryloyloxy group, the compound being represented by Formula (I)

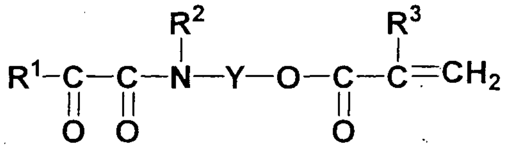 (I)

wherein Y is a $C_{1-6}$ straight-chain alkylene group which may have a $C_{1-6}$ organic group as a substituent; $R^1$ is a $C_{1-16}$ organic group; $R^2$ is a hydrogen atom or a $C_{1-8}$ organic group; and $R^3$ is a hydrogen atom or a methyl group.

2. A compound according to item 1, wherein Y is an ethylene group which may have a $C_{1-6}$ organic group as a substituent.

3. A compound according to item 1, wherein Y is a straight-chain propylene group which may have a $C_{1-6}$ organic group as a substituent.

4. A compound according to item 1, wherein Y is an ethylene group, $R^1$ is a methyl group, $R^2$ is a hydrogen atom, and $R^3$ is a hydrogen atom or a methyl group.

5. A compound according to item 1, wherein Y is an ethylene group, $R^1$ is a methyl group, $R^2$ is a $C_{1-4}$ alkyl group, and $R^3$ is a hydrogen atom or a methyl group.

6. A compound according to item 5, wherein $R^2$ is a methyl group.

7. A process for producing a monomer compound represented by Formula (I), the process comprising the steps of:

(1) reacting a compound represented by Formula (II)

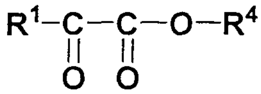 (II)

wherein $R^1$ is a $C_{1-16}$ organic group, and $R^4$ is a hydrogen atom or a $C_{1-18}$ organic group, with at least one compound selected from the group consisting of alcohols, thiols, acetals, and orthoesters, to obtain a compound represented by Formula (III)

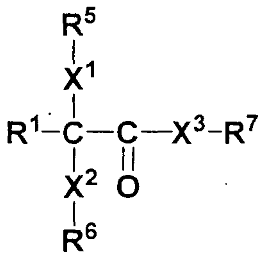 (III)

wherein $X^1$, $X^2$, and $X^3$ are each independently an oxygen atom or a sulfur atom; $R^5$ and $R^6$ are each independently a $C_{1-18}$ organic group; $R^7$ is a hydrogen atom or a $C_{1-18}$ organic group; two or three organic groups selected from $R^5$, $R^6$, and $R^7$ may be bonded to each other to form a heterocyclic ring; and $R^1$ is as defined above;
(2) reacting the compound of Formula (III) with a compound represented by Formula (IV)

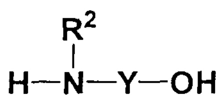 (IV)

wherein Y is a $C_{1-6}$ straight-chain alkylene group which may have a $C_{1-6}$ organic group as a substituent; and $R^2$ is a hydrogen atom or a $C_{1-8}$ organic group, to obtain a compound represented by Formula (V)

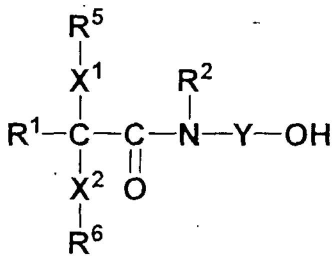

(V)

wherein $X^1$, $X^2$, Y, $R^1$, $R^2$, $R^5$, and $R^6$ are as defined above;

(3) reacting the compound of Formula (V) with a (meth)acrylic acid ester, (meth)acrylic acid, (meth)acrylic acid halide, or (meth)acrylic anhydride, to obtain a compound represented by Formula (VI)

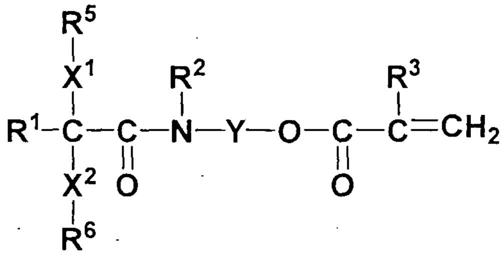

(VI)

wherein $X^1$, $X^2$, Y, $R^1$, $R^2$, $R^5$, and $R^6$ are as defined above; and $R^3$ is a hydrogen atom or a methyl group; and

(4) reacting the compound of Formula (VI) with water, in a dilute acid or in the presence of an acid compound, to obtain an α-oxoamide group- and (meth)acryloyloxy group-containing monomer compound represented by Formula (I)

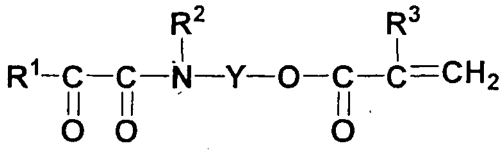

(I)

wherein Y, $R^1$, $R^2$, and $R^3$ are as defined above.

8. A process according to item 7, wherein, in Step 3, the compound of Formula (V) is reacted with a (meth)acrylic acid ester in the presence of a tin catalyst, to obtain the compound of Formula (VI).

9. A process according to item 8, wherein the tin catalyst is at least one organic tin compound selected from the group consisting of dialkyltin oxides, dialkyltin dicarboxylates, monoalkyltin tricarboxylates, monoalkyltin oxides, tin dicarboxylates, and organic distannoxanes.

10. The process according to item 9, wherein the tin catalyst is a dialkyltin oxide.

11. The process according to item 10, wherein the dialkyltin oxide is a dioctyltin oxide.

12. A polymer obtained by homopolymerizing the monomer compound according to item 1 or by copolymerizing the monomer compound with a vinyl monomer or monomers other than the compound, the polymer having α-oxoamide groups represented by Formula (VII)

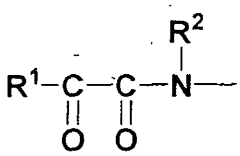

(VII)

wherein $R^1$ is a $C_{1-16}$ organic group, and $R^2$ is a hydrogen atom or a $C_{1-8}$ organic group.
13. The polymer according to item 12, wherein the molar concentration of α-oxoamide groups of Formula (VII) in the polymer is 0.05 to 5.5 mol/kg.
14. The polymer according to item 12, wherein the homopolymerization or copolymerization is carried out in an organic solvent.
15. The polymer according to item 12, wherein the homopolymerization or copolymerization is carried out in an emulsified state in water.
16. The polymer according to item 12, the polymer further containing at least one anionic group, selected from the group consisting of carboxylate groups, phosphate groups, and sulfonate groups.
17. The polymer according to item 12, the polymer further containing at least one nonionic hydrophilic group selected from the group consisting of polyoxyethylene groups, polyoxypropylene groups, and polyoxyethylene polyoxypropylene groups.
18. An aqueous curable composition comprising:

(A) the polymer according to item 12, and
(B) a compound containing, per molecule, at least two crosslinkable groups of at least one kind selected from the group consisting of primary amino groups, secondary amino groups, and ammonium groups.

19. An aqueous curable composition according to item 18, wherein the primary amino group or groups and/or secondary amino group or groups in the compound (B) are neutralized with a Brönsted acid.
20. An aqueous curable composition according to item 18, wherein the compound (B) is at least one compound selected from the group consisting of (polyamino)alkanes, (polyamino)polyether compounds, poly(ethyleneimine)s, and poly(aminoalkene)s.
21. An aqueous curable composition according to item 18, wherein the compound (B) is a cationic resin produced using, as starting materials, an amine compound and a compound having at least two glycidyl groups per molecule.
22. An aqueous curable composition according to item 18, wherein the compound (B) further contains at least one nonionic hydrophilic group selected from the group consisting of monoalkyl polyoxyethylene groups, monoalkyl polyoxypropylene groups, and monoalkyl polyoxyethylene polyoxypropylene groups.
23. An aqueous curable composition according to item 18, wherein the molar concentration of α-oxoamide groups of Formula (VII) in the polymer (A) is 0.05 to 5.5 mol/kg; and wherein the molar concentration of crosslinkable groups in the compound (B) is 0.05 to 35 mol/kg.
24. An aqueous curable composition according to item 18, wherein the polymer (A) further contains carboxy and carboxylate groups in a total molar concentration of 0.05 to 2 mol/kg.
25. An aqueous curable composition according to item 18, wherein the ratio of the polymer (A) to the compound (B) is such that the ratio of α-oxoamide groups of Formula (VII) in the polymer (A) to crosslinkable groups in the compound (B) is (the number of moles of the α-oxoamide groups)/(number of moles of moles of the crosslinkable groups) = 0.3 to 3.
26. An aqueous curable composition according to item 18, wherein the polymer (A) further contains carboxy and carboxylate groups; and wherein the polymer (A) and the amine compound (B) are contained in such a ratio that the ratio of the total number of moles of the carboxy and carboxylate groups to the number of moles of crosslinkable groups in the compound (B) is (the total number of moles of the carboxy and carboxylate groups)/(the number of moles of the crosslinkable groups) = 0.05 to 10.
27. A method for forming a coating film, the method comprising applying an aqueous curable composition according to item 18 by electrodeposition.

As used herein, "(meth)acryloyloxy" means acryloyloxy or methacryloyloxy.

α-Oxoamide group-and (meth)acryloyloxy group-containing monomer compound

The α-oxoamide group- and (meth)acryloyloxy group-containing monomer compound of the present invention is represented by Formula (I)

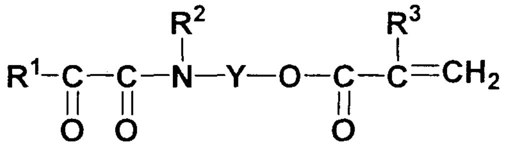 (I)

wherein Y is a $C_{1-6}$ straight-chain alkylene group which may have a $C_{1-6}$ organic group as a substituent; $R^1$ is a $C_{1-16}$ organic group; $R^2$ is a hydrogen atom or a $C_{1-8}$ organic group; and $R^3$ is a hydrogen atom or a methyl group.

In the compound of Formula (I), the $C_{1-6}$ straight-chain alkylene group represented by Y is ethylene, propylene, butylene, heptylene, or hexylene. The $C_{1-6}$ organic group which may be possessed by the straight-chain alkylene group may be, for example, methyl, ethyl, isopropyl, or like alkyl group; methoxy, ethoxy, or like alkoxy group; or the like.

The $C_{1-16}$ organic group represented by $R^1$ may be, for example, methyl, ethyl, propyl, butyl, pentyl, hexyl, heptyl, octyl, nonyl, decyl, undecyl, dodecyl, tridecyl, tetradecyl, pentadecyl, hexadecyl, or an alkyl group that is isomeric therewith; methoxymethyl, ethoxymethyl, butoxyethyl, methoxypropyl, propoxypropyl, or like alkoxyalkyl group; or the like. When $R^1$ has 17 or more carbon atoms, polymers of the compound of Formula (I) may have low compatibility with other polymers.

The $C_{1-8}$ organic group represented by $R^2$ may be, for example, methyl, ethyl, propyl, butyl, pentyl, hexyl, heptyl, octyl, or an alkyl group that is isomeric therewith; methoxymethyl, ethoxymethyl, propoxymethyl, butoxymethyl, butoxyethyl, or like alkoxyalkyl group; or the like. When $R^2$ has 9 or more carbon atoms, the production of the compound of Formula (I) may require a long time.

Production process for monomer compound

The production process for the monomer compound represented by Formula (I) is not limited. A process that comprises the following Steps 1 to 4 and uses a compound of Formula (II) as a starting material is simple, and thus is preferable as a process for producing the monomer compound of Formula (I).

Step 1

Step 1 is a step of reacting a compound represented by Formula (II)

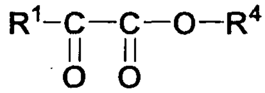 (II)

wherein $R^1$ is as defined above, and $R^4$ is a hydrogen atom or a $C_{1-18}$ organic group; with at least one compound selected from the group consisting of alcohols, thiols, acetals, and orthoesters, to thereby obtain a compound represented by Formula (III)

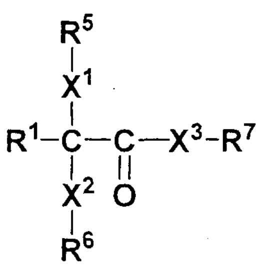 (III)

wherein $X^1$, $X^2$, and $X^3$ are each independently an oxygen atom or a sulfur atom, $R^5$ and $R^6$ are each independently a $C_{1-18}$ organic group, $R^7$ is a hydrogen atom or a $C_{1-18}$ organic group, two or three organic groups selected from $R^5$, $R^6$ and $R^7$ may be bonded to each other to form a heterocyclic ring, and $R^1$ is as defined above.

In the compound of Formula (II) used in Step 1, examples of the $C_{1-18}$ organic group represented by $R^4$ include,

in addition to the $C_{1-16}$ organic groups mentioned for $R^1$, heptadecyl, octadecyl, and alkyl groups that are isomeric therewith; butoxyethyl, (2-methoxy)propyl, and like alkoxyalkyl groups; etc. From the viewpoint of high reactivity, $R^4$ is preferably a hydrogen atom or a $C_{1-8}$ organic group, and more preferably a hydrogen atom or a $C_{1-4}$ organic group. When the organic group represented by $R^4$ has 19 or more carbon atoms, the production of the compound of Formula (III) is likely to require a greatly prolong time, resulting in low productivity.

Preferable alcohols to be reacted with the compound of Formula (II) in Step 1 include $C_{1-18}$ hydroxy-containing compounds. Examples of such hydroxy-containing compounds include methanol, ethanol, propanol, butanol, pentanol, hexanol, cyclohexanol, heptanol, octanol, nonanol, decanol, undecanol, dodecanol, tridecanol, tetradecanol, pentadecanol, hexadecanol, heptadecanol, octadecanol, isomers thereof, and like monools; propylene glycol monomethyl ether, propylene glycol monopropyl ether, ethylene glycol monobutyl ether, and like ether group-containing monools; ethylene glycol, propylene glycol, 1,3-propanediol, 2,2-dimethyl-1,3-propanediol, 2-ethyl-2-butyl-1,3-propanediol, 2-ethyl-1,3-hexanediol, and like diols; glycerol, trimethylolethane, trimethylolpropane, trimethyloloctane, and like triols; etc. Such compounds can be used singly or in combination.

Preferable thiols to be reacted with the compound of Formula (II) in Step 1 include $C_{1-18}$ mercapto group-containing compounds. Such mercapto group-containing compounds include, for example, methanethiol, ethanethiol, propanethiol, butanethiol, pentanethiol, hexanethiol, cyclohexanethiol, heptanethiol, octanethiol, nonanethiol, decanethiol, undecanethiol, dodecanethiol, tridecanethiol, tetradecanethiol, pentadecanethiol, hexadecanethiol, heptadecanethiol, octadecanethiol, isomers thereof, and like monothiols; 1,2-ethanedithiol, 1,3-propanedithiol, and like dithiols; etc. Such compounds can be used singly or in combination.

Suitable acetals to be reacted with the compound of Formula (II) in Step 1 include compounds obtained by dehydration condensation of $C_{1-18}$ hydroxy-containing compounds with ketones under acidic catalytic conditions. Usable ketones are not limited, but $C_{3-9}$ ketones are preferable. Examples of such ketones include acetone, methyl ethyl ketone, diethyl ketone, methyl isobutyl ketone, methyl t-butyl ketone, cyclohexanone, isophorone, etc.

Specific examples of usable acetals include 2,2-dimethoxypropane, 2,2-diethoxypropane, 2,2-dibutoxypropane, 2,2-dimethoxybutane, 2,2-diethoxybutane, 2,2-dibutoxybutane, 2,2-dimethoxy-4-methylpentane, 2,2-diethoxy-4-methylpentane, 2,2-dibutoxy-4-methylpentane, and like noncyclic acetals; 2,2-dimethyl-1,3-dioxolane, 2,2-dimethyl-4-methyl-1,3-dioxolane, 2-ethyl-2-methyl-1,3-dioxolane, 2-ethyl-2-methyl-4-methyl-1,3-dioxolane, 2-isobutyl-2-methyl-1,3-dioxolane, 2-isobutyl-2-methyl-4-methyl-1,3-dioxolane, 2,2-dimethyl-1,3-dioxane, 2,2-dimethyl-4-methyl-1,3-dioxane, 2-ethyl-2-methyl-1,3-dioxane, 2-ethyl-2-methyl-4-methyl-1,3-dioxane, 2-isobutyl-2-methyl-1,3-dioxane, 2-isobutyl-2-methyl-4-methyl-1,3-dioxane, and like cyclic acetals; etc. Among these, 2,2-dimethoxypropane, 2,2-dimethoxybutane, 2,2-diethoxypropane, 2,2-diethoxybutane, 2,2-dimethyl-1,3-dioxolane, and 2,2-dimethyl-4-methyl-1,3-dioxolane are industrially readily available and are thus preferable. Such acetals can be used singly or in combination.

Orthoesters to be reacted with the compound of Formula (II) in Step 1 are not limited, but from the viewpoint of availability, those having 4 to 14 carbon atoms are preferable. Such orthoesters include, for example, trimethyl orthoformate, triethyl orthoformate, triisopropyl orthoformate, tributyl orthoformate, trimethyl orthoacetate, triethyl orthoacetate, triisopropyl orthoacetate, tributyl orthoacetate, etc., among which trimethyl orthoformate, triethyl orthoformate, trimethyl orthoacetate, and triethyl orthoacetate are inexpensive and thus are more preferable. Such orthoesters can be used singly or in combination.

The amount of at least one compound selected from the group consisting of alcohols, thiols, acetals, and orthoesters used in Step 1 is not limited, and is preferably about 1 to about 10 mol, and more preferably about 1.05 to about 5 mol, per mol of the compound of Formula (II).

The reaction of Step 1 is carried out by mixing the compound of Formula (II) with at least one compound selected from the group consisting of alcohols, thiols, acetals, and orthoesters. The reaction is usually performed for about 10 minutes to about 30 hours. In order to prevent coloring of the product, the reaction may be carried out in an atmosphere of an inert gas, such as nitrogen or the like. The reaction can be performed with heating, under pressure, with addition of a small amount of acid catalyst, and/or using a solvent, as required. Further, the water, ketone, ester, alcohol, thiol, etc. that are formed in the reaction system may be removed from the system to promote the reaction. It is usually preferable to carry out heating at about 30 to about 200°C, but too low a heating temperature shows only a small reaction promoting effect whereas too high a heating temperature makes it likely that the product will be colored. Therefore, heating temperatures of about 40 to about 180°C are more preferable.

Usable acid catalysts are not limited, and include, for example, dilute sulfuric acid, dilute hydrochloric acid, phosphoric acid, and like inorganic Brönsted acids; paratoluenesulfonic acid, methanesulfonic acid, and like organic sulfonic acids; monoalkyl phosphates, dialkyl phosphates, and like phosphoric acid esters; formic acid, trifluoroacetic acid, oxalic acid, and like carboxylic acids; trimethylsilyl halide, boron trifluoride complexes, titanium tetraalkoxides, titanium tetrahalides, dialkyltin oxides, dialkyltin dicarboxylates, monoalkyltin tricarboxylates, tin dicarboxylates, tin tetrachloride, zirconium tetraalkoxides, zirconium tetrahalide, zinc salts, chromium salts, manganese salts, iron salts, copper salts, cerium salts, scandium salts, yttrium salts, lanthanoid salts, lead compounds, bismuth compounds, antimony compounds, metal triflates, metal tetrafluoroborate salts, metal acetylacetonate salts, and like Lewis acids; clay, activated

clay, acid clay, silica, alumina, zeolite, cation exchange resins, and like solid acid catalysts; etc.

Preferable cation exchange resins include ion exchange resins having functional groups such as sulfonic acid groups, phosphoric acid groups, carboxy groups, etc., among which ion exchange resins having sulfonic acid groups or phosphoric acid groups are particularly preferable. Examples of commercially available cation exchange resins include "Amberlyst 15J WET", "Amberlyst 15 DRY", "Amberlyst 16 WET", and "Amberlyst 31 WET" (tradenames of Rohm & Haas Co.); "RCP-160M", "RCP-150H", "RCP-170H", and "PK-216" (tradenames of Mitsubishi Chemical Corp.); "Dowex 50W" (tradename of Dow Chemical Co.); etc.

The amount of acid catalyst to be added is not limited, but when the acid catalyst is an inorganic acid, organic sulfonic acid, phosphoric acid ester, carboxylic acid, or Lewis acid, the amount thereof is preferably about 0.01 to about 10 mol per mol of the compound represented by Formula (II). When the acid catalyst is a solid acid catalyst, the amount thereof is preferably about 0.1 to about 500 parts by weight per 100 parts by weight of the compound represented by Formula (II). The solid acid catalyst can be separated by filtration after the reaction.

Known organic solvents can be used as reaction solvents. The amount of solvent to be used is not limited, but is preferably not more than ten times the weight of the compound represented by Formula (II), since use of too large an amount of solvent is uneconomical.

The water, ketone, ester, alcohol, thiol, etc. that are formed in the reaction system can be removed from the system at atmospheric pressure or under elevated or reduced pressure, optionally with heating and/or while introducing a gas, such as air, nitrogen, or the like.

In the reaction of Step 1, an antioxidant may be added to the system in order to prevent formation of peroxides. The antioxidant to be used is not limited, and may be, for example, at least one member selected from benzoquinone and like quinone compounds; hydroquinone, hydroquinone monomethyl ether, di-t-butyl methyl phenol, and like phenolic compounds; phenylenediamine compounds, phenothiazine, and like amine compounds; N-oxyl compounds; nitroso compounds; nitrogen oxides; etc. When using antioxidant(s), the amount thereof is not limited, but is preferably about 10 to about 10,000 ppm, and more preferably about 100 to about 5,000 ppm, relative to the total amount of the compound represented by Formula (II) and compound(s) selected from the group consisting of alcohols, thiols, acetals, and orthoesters.

In the compound represented by Formula (III) obtained by the reaction of Step 1, $X^1$, $X^2$, $X^3$, $R^5$, $R^6$, and $R^7$ are determined depending on the kind of alcohol, thiol, acetal, and/or orthoester used in the reaction, and when alcohols, thiols, acetals, and/or orthoesters of the above-mentioned kinds are used, $X^1$, $X^2$, and $X^3$ are each independently an oxygen atom or a sulfur atom; $R^5$ and $R^6$ are each independently a $C_{1-18}$ organic group; $R^7$ is a hydrogen atom or a $C_{1-18}$ organic group; and two or three organic groups selected from $R^5$, $R^6$, and $R^7$ may be bonded to each other to form a heterocyclic ring containing two or more hetero atoms selected from $X^1$, $X^2$, and $X^3$. Examples of such heterocyclic rings include cyclic acetals, cyclic thioacetals,cyclic orthoesters, etc. $R^1$ is as defined above.

Examples of the compound represented by Formula (III) include 2,2-dialkoxyalkanoic acid esters, 2-alkyl-2-alkoxycarbonyl-1,3-dioxolane, 2-alkyl-2-alkoxycarbonyl-1,3-dioxane, 2,2-di(alkylthio)alkanoic acid esters, 2,2-di(alkylthio)thioalkanoic acid esters, 2-alkyl-2-alkoxycarbonyl-1,3-dithiolane, 2-alkyl-2-alkoxycarbonyl-1,3-dithiane, 2-alkyl-2-(alkylthio)carbonyl-1,3-dithiolane, 2-alkyl-2-(alkylthio)carbonyl-1,3-dithiane, etc.

In the reaction of Step 1, the compound of Formula (III) is obtained in the form of a solution in many cases. A compound with higher purity can be obtained by concentrating the solution. Further, distillation purification, extraction operation, and/or washing with water can be performed as required, to achieve even higher purity.

When an acid catalyst is used in the reaction of Step 1, some or all of the acid catalyst may be neutralized with a base or removed by adsorption using an adsorbent, before concentration, distillation purification, extraction operation, or washing of the solution containing the compound represented by Formula (III). Usable bases are not limited, and include, for example, metal hydroxides, metal oxides, alkoxides, ammonia, amines, metal carboxylates, etc. Usable adsorbents are not limited, and include, for example, clay, acid clay, activated clay, alumina, silica, zeolite, ion exchange resins, basic inorganic compounds, etc.

Step 2

Step 2 is a step of reacting the compound of Formula (III) obtained in Step 1 with a compound represented by Formula (IV)

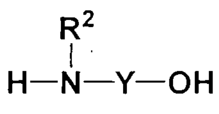

(IV)

wherein Y and $R^2$ are as defined above, to obtain a compound represented by Formula (V)

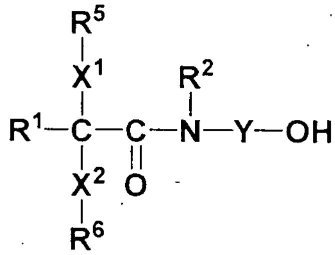

(V)

wherein Y, $X^1$, $X^2$, $R^1$, $R^2$, $R^5$, and $R^6$ are as defined above.

In Step 2, the compound of Formula (III) may be purified before being reacted with the compound of Formula (IV), or a solution containing the compound of Formula (III) may be reacted, without being purified, with the compound of Formula (IV). For purification, concentration, extraction, washing, filtration, and/or other procedures can be performed. When an acid catalyst is used in Step 1, some or all of the acid catalyst may be neutralized with a base or removed by adsorption using an adsorbent, before mixing the compound of Formula (III) with the compound of Formula (IV).

In the compound represented by Formula (IV), $R^2$ is preferably a hydrogen atom; methyl, ethyl, propyl, butyl, pentyl, hexyl, heptyl, octyl, or an alkyl group that is isomeric therewith; an oxygen atom-containing $C_{1-8}$ organic group; or the like; and more preferably a hydrogen atom or a $C_{1-4}$ alkyl group from the viewpoint of availability of the compound. When $R^2$ is an organic group having 9 or more carbon atoms, the production time for the compound of Formula (V) may be prolonged, resulting in low productivity.

Further, in the compound of Formula (IV), when the substituent possessed by the $C_{1-6}$ straight-chain alkylene group represented by Y is an organic group having 7 or more carbon atoms, the production time for the compound of Formula (V) may require a greatly prolonged time, resulting in poor productivity.

Specific examples of the compound of Formula (IV) include 2-aminoethanol, 2-(methylamino)ethanol, 2-(ethylamino)ethanol, 2-(isopropylamino)ethanol, 2-(butylamino)ethanol, 2-(t-butylamino)ethanol, and like 2-aminoethanol derivatives; 1-amino-2-propanol, 1-(methylamino)-2-propanol, 1-(ethylamino)-2-propanol, 1-(isopropylamino)-2-propanol, 1-(butylamino)-2-propanol, 1-(t-butylamino)-2-propanol, and like 1-amino-2-propanol derivatives; 2-amino-1-propanol, 2-amino-2-methyl-1-propanol, 2-amino-1-butanol, 2-amino-1-pentanol, 2-amino-1-hexanol, and like 2-amino-1-alkanol derivatives; 1-amino-2-butanol, 1-(methylamino)-2-butanol, 1-(ethylamino)-2-butanol, 1-(isopropylamino)-2-butanol, 1-(butylamino)-2-butanol, 1-(t-butylamino)-2-butanol, and like 1-aminobutanol derivatives; 3-amino-l-propanol, 4-amino-1-butanol, 6-amino-1-hexanol, and like ω-amino-1-alkanol derivatives; etc. Among such compounds, 2-aminoethanol derivatives in which Y is an ethylene group are preferable from the viewpoint of reduction of the production time for the compound of Formula (V).

The amount of the compound of Formula (IV) reacted with the compound of Formula (III) is not limited, but is preferably about 1 to about 3 mol, and more preferably about 1.05 to about 1.5 mol, per mol of the compound of Formula (III).

The reaction of the compound of Formula (III) with the compound of Formula (IV) is carried out by mixing these compounds, and is usually continued for about 10 minutes to about 30 hours. The reaction may be performed in an inert gas atmosphere to prevent coloring of the product. Further, if necessary, the reaction can be promoted by heating, elevating the pressure, adding a catalyst, using a solvent, removing alcohols formed, and/or other methods.

It is usually preferable that the reaction temperature be about 30 to about 230°C, but reaction temperatures of about 40 to about 180°C are more preferable since too low a reaction temperature produces only a small reaction promoting effect, whereas too high a reaction temperature is likely to color the product.

Acid catalysts and base catalysts are both usable, with acid catalysts being preferable. Usable base catalysts are not limited, and include, for example, metal hydroxides, metal alkoxides, metal oxides, ammonia, amines, metal carboxylates, metal phenoxides, basic organic onium salts, anion exchange resins containing hydroxide ions or carboxylate ions, etc. The amount of base catalyst to be added is not limited, but when using a metal hydroxide, metal alkoxide, metal oxide, ammonia, amine, metal carboxylate, metal phenoxide, basic organic onium salt, or the like, the amount thereof is preferably about 0.01 to about 10 mol per mol of the compound of Formula (III), and when using an anion exchange resin, the amount thereof is preferably about 0.1 to about 500 parts by weight per 100 parts by weight of the compound of Formula (III). The anion exchange resin can be separated by filtration after the reaction.

Known organic solvents can be used as reaction solvents, and solvents with relatively high polarity are preferable. The amount of solvent to be used is not limited, but is preferably not more than about 10 times the weight of the compound of Formula (III), since use of too large an amount of solvent is uneconomical. The alcohol formed in the

reaction system can be removed from the system at atmospheric pressure or under reduced pressure, optionally with heating and/or while introducing a gas, such as air, nitrogen, or the like. When introducing a gas, an inert gas is preferable to prevent coloring of the product, and nitrogen is preferable from an economical viewpoint.

In the reaction of Step 2, in order to prevent formation of peroxides, an antioxidant may be added to the reaction system. The antioxidant is not limited, and may be, for example, at least one of the above-mentioned antioxidants. When adding antioxidant(s), the amount thereof is not limited, but is preferably about 10 to about 10,000 ppm, and more preferably about 100 to about 5,000 ppm, relative to the total amount of the compounds of Formula (III) and Formula (IV).

The compound represented by Formula (V) is obtained by reacting the compound of Formula (III) with the compound of Formula (IV). $X^1$, $X^2$, $R^1$, $R^2$, $R^5$, and $R^6$ in Formula (V) are determined depending on the structures of the compounds of Formula (III) and Formula (IV) used in the reaction.

Examples of the compound of Formula (V) include N-(2-hydroxyalkyl)-2,2-dialkoxyalkanoic acid amides, N-alkyl-N-(2-hydroxyalkyl)-2,2-dialkoxyalkanoic acid amides, 2-alkyl-2-(2-hydroxyalkyl)aminocarbonyl-1,3-dioxolanes, 2-alkyl-2-(N-alkyl-N-(2-hydroxyalkyl)amino)carbonyl-1,3-dioxolanes, 2-alkyl-2-(2-hydroxyalkyl)aminocarbonyl-1,3-dioxanes, 2-alkyl-2-(N-alkyl-N-(2-hydroxyalkyl)amino)carbonyl-1,3-dioxanes, N-(3-hydroxyalkyl)-2,2-dialkoxyalkanoic acid amides, N-alkyl-N-(3-hydroxyalkyl)-2,2-dialkoxyalkanoic acid amides, 2-alkyl-2-(3-hydroxyalkyl)aminocarbonyl-1,3-dioxolanes, 2-alkyl-2-(N-alkyl-N-(3-hydroxyalkyl)amino)carbonyl-1,3-dioxolanes, 2-alkyl-2-(3-hydroxyalkyl)aminocarbonyl-1,3-dioxanes, 2-alkyl-2-(N-alkyl-N-(3-hydroxyalkyl)amino)carbonyl-1,3-dioxanes, N-(4-hydroxyalkyl)-2,2-dialkoxyalkanoic acid amides, N-alkyl-N-(4-hydroxyalkyl)-2,2-dialkoxyalkanoic acid amides, 2-alkyl-2-(4-hydroxyalkyl)aminocarbonyl-1,3-dioxolanes, 2-alkyl-2-(N-alkyl-N-(4-hydroxyalkyl)amino)carbonyl-1,3-dioxolanes, 2-alkyl-2-(4-hydroxyalkyl)aminocarbonyl-1,3-dioxanes, 2-alkyl-2-(N-alkyl-N-(4-hydroxyalkyl)amino)carbonyl-1,3-dioxanes, N-(5-hydroxyalkyl)-2,2-dialkoxyalkanoic acid amides, N-alkyl-N-(5-hydroxyalkyl)-2,2-dialkoxyalkanoic acid amides, 2-alkyl-2-(5-hydroxyalkyl)aminocarbonyl-1,3-dioxolanes, 2-alkyl-2-(N-alkyl-N-(5-hydroxyalkyl)amino)carbonyl-1,3-dioxolanes, 2-alkyl-2-(5-hydroxyalkyl)aminocarbonyl-1,3-dioxanes, 2-alkyl-2-(N-alkyl-N-(5-hydroxyalkyl)amino)carbonyl-1,3-dioxanes, N-(6-hydroxyalkyl)-2,2-dialkoxyalkanoic acid amides, N-alkyl-N-(6-hydroxyalkyl)-2,2-dialkoxyalkanoic acid amides, 2-alkyl-2-(6-hydroxyalkyl)aminocarbonyl-1,3-dioxolaries, 2-alkyl-2-(N-alkyl-N-(6-hydroxyalkyl)amino)carbonyl-1,3-dioxolanes, 2-alkyl-2-(6-hydroxyalkyl)aminocarbonyl-1,3-dioxanes, 2-alkyl-2-(N-alkyl-N-(6-hydroxyalkyl)amino)carbonyl-1,3-dioxanes, N-(2-hydroxyalkyl)-2,2-di(alkylthio)alkanoic acid amides, N-alkyl-N-(2-hydroxyalkyl)-2,2-di(alkylthio)alkanoic acid amides, 2-alkyl-2-(2-hydroxyalkyl)aminocarbonyl-1,3-dithiolanes, 2-alkyl-2-(N-alkyl-N-(2-hydroxyalkyl)amino)carbonyl-1,3-dithiolanes, 2-alkyl-2-(2-hydroxyalkyl)aminocarbonyl-1,3-dithianes, 2-alkyl-2-(N-alkyl-N-(2-hydroxyalkyl)amino)carbonyl-1,3-dithianes, N-(3-hydroxyalkyl)-2,2-di(alkylthio)alkanoic acid amides, N-alkyl-N-(3-hydroxyalkyl)-2,2-di(alkylthio)alkanoic acid amides, 2-alkyl-2-(3-hydroxyalkyl)aminocarbonyl-1,3-dithiolanes, 2-alkyl-2-(N-alkyl-N-(3-hydroxyalkyl)amino)carbonyl-1,3-dithiolanes, 2-alkyl-2-(3-hydroxyalkyl)aminocarbonyl-1,3-dithianes, 2-alkyl-2-(N-alkyl-N-(3-hydroxyalkyl)amino)carbonyl-1,3-dithianes, etc.

In the reaction of Step 2, the compound of Formula (V) is obtained in the form a solution in many cases. A highly pure compound can be obtained by concentrating the solution. Further, if necessary, distillation purification, recrystallization, extraction operation, washing with water, and/or other procedures can be performed to obtain a compound with higher purity.

When a base catalyst is used in Step 2, some or all of the base catalyst may be neutralized with an acid or removed by adsorption using an adsorbent, before concentration, distillation purification, recrystallization, extraction operation or washing of the solution containing the compound of Formula (V). Usable acids are not limited and include, for example, dilute sulfuric acid, dilute hydrochloric acid, phosphoric acid, and like inorganic Brönsted acids; paratoluene sulfonic acid, methanesulfonic acid, and like organic sulfonic acids; monoalkylphosphoric acids, dialkylphosphoric acids, and like phosphoric acid esters; formic acid, trifluoroacetic acid, oxalic acid, and like carboxylic acids; metal-containing compounds with Lewis acidity; clay, silica, zeolite, cation exchange resins, and like solid acid catalysts; etc. Usable adsorbents are not limited, and include, for example, acid clay, activated clay, alumina, silica, zeolite, basic inorganic compounds, ion exchange resins, etc.

Step 3

Step 3 is a step of reacting the compound of Formula (V) obtained in Step 2 with a (meth)acrylic acid ester, (meth)acrylic acid, (meth)acrylic acid halide, or (meth)acrylic anhydride, to obtain a compound represented by Formula (VI)

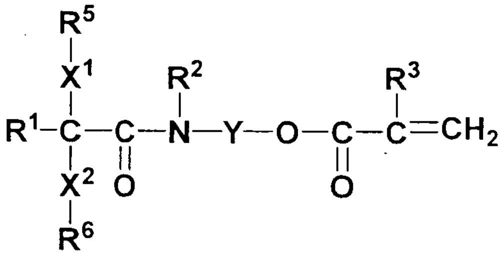

(VI)

wherein $X^1$, $X^2$, Y, $R^1$, $R^2$, $R^5$, and $R^6$ are as defined above; and $R^3$ is a hydrogen atom or a methyl group.

In Step 3, the compound represented by Formula (V) may be purified before being reacted with a (meth)acrylic acid ester, (meth)acrylic acid, (meth)acrylic acid halide, or (meth)acrylic anhydride, or a solution containing the compound of formula (V) may be reacted, without being purified, with a (meth)acrylic acid ester, (meth)acrylic acid, (meth)acrylic acid halide, or (meth)acrylic anhydride. For purification, concentration, liquid-liquid separation, washing, filtration, and/or other procedures can be performed. When a catalyst is used in the reaction of the compound of Formula (III) with the compound of Formula (IV), some or all of the catalyst may be neutralized or removed by adsorption using an adsorbent, before mixing the compound of Formula (V) with a (meth)acrylic acid ester, (meth)acrylic acid, (meth)acrylic acid halide, or (meth)acrylic anhydride.

The compound represented by Formula (VI) is an ester of the compound of Formula (V) with (meth)acrylic acid. $X^1$, $X^2$, $R^1$, $R^2$, $R^3$, $R^5$, and $R^6$ in Formula (VI) are determined depending on the structures of the compound of Formula (V) and the (meth)acrylic acid ester, (meth)acrylic acid, (meth)acrylic acid halide, or (meth)acrylic anhydride.

Examples of the compound represented by Formula (VI) include N-(2-((meth)acryloyloxy)alkyl)-2,2-dialkoxyalkanoic acid amides, N-alkyl-N-(2-((meth)acryloyloxy)alkyl)-2,2-dialkoxyalkanoic acid amides, 2-alkyl-2-(2-((meth)acryloyloxy)alkyl)aminocarbonyl-1,3-dioxolanes, 2-alkyl-2-(N-alkyl-N-(2-((meth)acryloyloxy)alkyl)amino)carbonyl-1,3-dioxolanes, 2-alkyl-2-(2-((meth)acryloyloxy)alkyl)aminocarbonyl-1,3-dioxanes, 2-alkyl-2-(N-alkyl-N-(2-((meth)acryloyloxy)alkyl)amino)carbonyl-1,3-dioxanes, N-(3-((meth)acryloyloxy)alkyl)-2,2-dialkoxyalkanoic acid amides, N-alkyl-N-(3-((meth)acryloyloxy)alkyl)-2,2-dialkoxyalkanoic acid amides, 2-alkyl-2-(3-((meth)acryloyloxy)alkyl)aminocarbonyl-1,3-dioxolanes, 2-alkyl-2-(N-alkyl-N-(3-((meth)acryloyloxy)alkyl)amino)carbonyl-1,3-dioxolanes, 2-alkyl-2-(3-((meth)acryloyloxy)alkyl)aminocarbonyl-1,3-dioxanes, 2-alkyl-2-(N-alkyl-N-(3-((meth)acryloyloxy)alkyl)amino)carbonyl-1,3-dioxanes, N-(4-((meth)acryloyloxy)alkyl)-2,2-dialkoxyalkanoic acid amides, N-alkyl-N-(4-((meth)acryloyloxy)alkyl)-2,2-dialkoxyalkanoic-acid amides, 2-alkyl-2-(4-((meth)acryloyloxy)alkyl)aminocarbonyl-1,3-dioxolanes, 2-alkyl-2-(N-alkyl-N-(4-((meth)acryloyloxy)alkyl)amino)carbonyl-1,3-dioxolanes, 2-alkyl-2-(4-((meth)acryloyloxy)alkyl)aminocarbonyl-1,3-dioxanes, 2-alkyl-2-(N-alkyl-N-(4-((meth)acryloyloxy)alkyl)amino)carbonyl-1,3-dioxanes, N-(5-((meth)acryloyloxy)alkyl)-2,2-dialkoxyalkanoic acid amides, N-alkyl-N-(5-((meth)acryloyloxy)alkyl)-2,2-dialkoxyalkanoic acid amides, 2-alkyl-2-(5-((meth)acryloyloxy)alkyl)aminocarbonyl-1,3-dioxolanes, 2-alkyl-2-(N-alkyl-N-(5-((meth)acryloyloxy)alkyl)amino)carbonyl-1,3-dioxolanes, 2-alkyl-2-(5-((meth)acryloyloxy)alkyl)aminocarbonyl-1,3-dioxanes, 2-alkyl-2-(N-alkyl-N-(5-((meth)acryloyloxy)alkyl)amino)carbonyl-1,3-dioxanes, N-(6-((meth)acryloyloxy)alkyl)-2,2-dialkoxyalkanoic acid amides, N-alkyl-N-(6-((meth)acryloyloxy)alkyl)-2,2-dialkoxy alkanoic acid amides, 2-alkyl-2-(6-((meth)acryloyloxy)alkyl)aminocarbonyl-1,3-dioxolanes, 2-alkyl-2-(N-alkyl-N-(6-((meth)acryloyloxy)alkyl)amino)carbonyl-1,3-dioxolanes, 2-alkyl-2-(6-((meth)acryloyloxy)alkyl)aminocarbonyl-1,3-dioxanes, 2-alkyl-2-(N-alkyl-N-(6-((meth)acryloyloxy)alkyl)amino)carbonyl-1,3-dioxane, N-(2-((meth)acryloyloxy)alkyl)-2,2-di(alkylthio)alkanoic acid amides, N-alkyl-N-(2-((meth)acryloyloxy)alkyl)-2,2-di(alkylthio)alkanoic acid amides, 2-alkyl-2-(2-((meth)acryloyloxy)alkyl)aminocarbonyl-1,3-dithiolanes, 2-alkyl-2-(N-alkyl-N-(2-((meth)acryloyloxy)alkyl)amino)carbonyl-1,3-dithiolanes, 2-alkyl-2-(2-((meth)acryloyloxy)alkyl)aminocarbonyl-1,3-dithianes, 2-alkyl-2-(N-alkyl-N-(2-((meth)acryloyloxy)alkyl)amino)carbonyl-1,3-dithiane, N-(3-((meth)acryloyloxy)alkyl)-2,2-di(alkylthio)alkanoic acid amides, N-alkyl-N-(3-((meth)acryloyloxy)alkyl)-2,2-di(alkylthio)alkanoic acid amides, 2-alkyl-2-(3-((meth)acryloyloxy)alkyl)aminocarbonyl-1,3-dithiolanes, 2-alkyl-2-(N-alkyl-N-(3-((meth)acryloyloxy)alkyl)amino)carbonyl-1,3-dithiolanes, 2-alkyl-2-(3-((meth)acryloyloxy)alkyl)aminocarbonyl-1,3-dithianes, 2-alkyl-2-(N-alkyl-N-(3-((meth)acryloyloxy)alkyl)amino)carbonyl-1,3-dithianes, etc.

The reaction of the compound of Formula (V) with a (meth)acrylic acid ester is carried out by transesterification. The (meth)acrylic acid ester to be used in the reaction is preferably a (meth)acrylic acid ester having a $C_{1-18}$ alkyl group, and, from the viewpoint of high reaction rate, it is more preferable to use a (meth)acrylic acid ester having a $C_{1-4}$ alkyl group and having a relatively low boiling point, such as methyl (meth)acrylate, ethyl (meth)acrylate, butyl (meth) acrylate, or the like. When the (meth)acrylic acid ester has an alkyl group having 19 or more carbon atoms, the reaction rate tends to be low.

The transesterification is performed at atmospheric pressure or under elevated or reduced pressure, usually with heating. The amount of (meth)acrylic acid ester is not limited, but is preferably about 1 to about 10 mol, and more

preferably about 1 to about 5 mol, per mol of the compound of Formula (V). Heating is preferably carried out at about 60 to about 180°C, but when the heating temperature is too low, the reaction rate becomes low, whereas when the heating temperature is too high, the product is likely to be polymerized. Therefore, heating temperatures of about 70 to about 150°C are more preferable. The reaction may be promoted by adding a catalyst, using a solvent, removing formed alcohols from the system, and/or other procedures.

The catalyst may be at least one member selected from the group consisting of Brönsted acid catalysts, base catalysts, and Lewis acid catalysts, among which Lewis acid catalysts are preferable since they have high catalytic performance. Usable Lewis acid catalysts are not limited, and include titanium tetraalkoxides, titanium tetrahalides, and like titanium compounds; dialkyltin oxides, dialkyltin dicarboxylates, monoalkyltin tricarboxylates, tin dicarboxylates, tin tetrachloride, stannoxane derivatives, and like tin compounds; zirconium tetraalkoxides, zirconium tetrahalides, and like zirconium compounds; zinc dicarboxylates, zinc halides, and like zinc compounds; lead compounds; bismuth compounds; antimony compounds; clay, acid clay, activated clay, silica, alumina, zeolite, cation exchange resins, and like solid acid catalysts; etc.

Among such catalysts, tin compounds are particularly preferable since use of a tin compound as a catalyst increases the purity of the compound of Formula (I) of the present invention. Specific examples of tin compounds include dioctyltin oxide, dibutyltin oxide, and like dialkyltin oxides; dioctyltin diacetate, dioctyltin dilaurate, dibutyltin diacetate, dibutyltin dilaurate, and like dialkyltin dicarboxylates; monooctyltin triacetate, monooctyltin trilaurate, monobutyltin triacetate, monobutyltin trilaurate, and like monoalkyltin tricarboxylates; monooctyltin oxide, monobutyltin oxide, and like monoalkyltin oxides; tin diacetate, tin dilaurate, and like tin carboxylates; tetraalkyldistannoxane derivatives, and like organic distannoxanes; etc. Among such tin compounds, dialkyltin oxides and organic distannoxanes are preferable. Further, dialkyltin oxides, such as dioctyltin oxide and the like, are particularly preferable since they are inexpensive.

The amount of catalyst to be added is not limited, but when using a catalyst other than solid acid catalysts, the amount thereof is preferably about 0.01 to about 10 mol per mol of the compound of Formula (V). When using a solid acid catalyst, the amount thereof is preferably about 0.1 to about 500 parts by weight per 100 parts by weight of the compound of Formula (V).

The reaction of Step 3 can be promoted by using a solvent, removing formed alcohols from the reaction system, and/or other methods. In such a case, known organic solvents are usable, and the amount of solvent to be used is not limited. Since use of solvent in too large an amount is uneconomical, the amount of solvent to be used is preferably not more than about 5 times the weight of the compound of Formula (V). The alcohol formed in the reaction system can be removed from the system at atmospheric pressure or under reduced or elevated pressure.

The reaction of the compound of Formula (V) with (meth)acrylic acid is carried out by dehydration condensation. The dehydration condensation reaction is usually performed with heating. It is usually preferable that the heating temperature be about 60 to about 230°C, but too low a heating temperature makes the reaction rate low, whereas too high a heating temperature makes it likely that the product will be polymerized. Therefore, heating temperatures of about 70 to about 170°C are more preferable. The amount of (meth)acrylic acid is not limited, but is preferably about 1 to about 10 mol, and more preferably about 1 to about 5 mol per mol of the compound of Formula (V). The reaction may be promoted by adding a catalyst, using a solvent, removing formed water from the reaction system, and/or other methods.

The catalyst is preferably a Brönsted acid catalyst and/or Lewis acid catalyst. Usable Brönsted acids are not limited, but from the viewpoint of high reaction rate, preferable Brönsted acids include dilute sulfuric acid, dilute hydrochloric acid, phosphoric acid, and like inorganic acids; paratoluene sulfonic acid, methanesulfonic acid, and like organic sulfonic acids; monoalkyl phosphates, dialkyl phosphates, and like phosphoric acid esters; etc. Usable Lewis acids are not limited, but from the viewpoint of high reaction rate, preferable Lewis acids include dialkyltin oxides, dialkyltin dicarboxylates, monoalkyltin tricarboxylates, tin dicarboxylates, tin tetrachloride, and like tin compounds; zirconium tetraalkoxides, zirconium tetrahalides, and like zirconium compounds; zinc dicarboxylates, zinc halides, and like zinc compounds; lead compounds; bismuth compounds; antimony compounds; clay, activated clay, acid clay, silica, alumina, zeolite, cation exchange resins, and like solid acid catalysts; etc. The amount of catalyst to be added is not limited, but when using a catalyst other than solid acid catalysts, the amount thereof is preferably about 0.01 to about 10 mol per mol of the compound represented by Formula (V), and when using a solid acid catalyst, the amount thereof is preferably about 0.1 to about 500 parts by weight per 100 parts by weight of the compound of Formula (V). The solvent to be used may be a known aprotic organic solvent. The amount of solvent to be used is not limited, but is preferably not more than about 5 times the weight of the compound of Formula (V), since use of solvent in too large an amount is uneconomical. The water formed in the reaction system can be removed from the system at atmospheric pressure or under reduced or elevated pressure.

The reaction of the compound of Formula (V.) with a (meth)acrylic acid halide is usually performed in the presence of a base. The base is used to neutralize the acid formed, and is not limited. Examples of usable bases include triethylamine, pyridine, sodium hydroxide, sodium carbonate, sodium hydrogencarbonate, etc. The amount of (meth) acrylic acid halide is preferably about 1 to about 2 mol per mol of the compound represented by Formula (V), and the amount of base is preferably about 1 to about 2 mol per mol of (meth)acrylic acid halide. The reaction is usually carried

out at about -70 to about 80°C, but too low a reaction temperature makes the reaction rate low, whereas too high a reaction temperature is likely to result in formation of byproducts. Therefore, it is preferable to carry out the reaction at about -30 to about 50°C. In that reaction, organic solvents and water are usable as solvents. When using water, the reaction system is likely to be a two-phase system. Therefore, the system is thoroughly stirred, and an onium salt or the like may be added to increase the reaction rate. The amount of solvent to be used is not limited, but is preferably not more than about 5 times the weight of the compound of Formula (V), since use of too large an amount of solvent is uneconomical.

The reaction of the compound of Formula (V) with (meth)acrylic anhydride is carried out by alcoholysis of the acid anhydride. The amount of (meth)acrylic anhydride is not limited, but is preferably about 1 to about 5 mol, and more preferably about 1 to about 3 mol, per mol of the compound of Formula (V). In this reaction, a catalyst may be used to promote the reaction. The catalyst may be any of Brönsted acid catalysts, base catalysts, and Lewis acid catalysts. Usable Brönsted acids are not limited and include, for example, dilute sulfuric acid, dilute hydrochloric acid, phosphoric acid, and like inorganic acids; paratoluene sulfonic acid, methanesulfonic acid, and like organic sulfonic acids; monoalkyl phosphates, dialkyl phosphates, and like phosphoric acid esters; etc. Examples of base catalysts include triethylamine, pyridine, sodium hydroxide, sodium carbonate, sodium hydrogencarbonate, etc. Examples of Lewis acid catalysts include titanium tetraalkoxides, titanium tetrahalides, and like titanium compounds; dialkyltin oxides, dialkyltin dicarboxylates, monoalkyltin tricarboxylates, tin dicarboxylates, tin tetrachloride, and like tin compounds; zirconium tetraalkoxides, zirconium tetrahalides, and like zirconium compounds; zinc dicarboxylates, zinc halides, and like zinc compounds; lead compounds; bismuth compounds; antimony compounds; clay, acid clay, activated clay, silica, alumina, zeolite, cation exchange resins, and like solid acid catalysts; etc. The amount of catalyst to be added is not limited, but when using a catalyst other than solid acid catalysts, the amount thereof is preferably about 0.01 to about 10 mol per mol of the compound of Formula (V), and when using a solid acid catalyst, the amount thereof is preferably about 0.1 to about 500 parts by weight per 100 parts by weight of the compound of Formula (V). Aprotic organic solvents can be used as reaction solvents. The amount of solvent to be used is not limited, but is preferably not more than about 5 times the weight of the compound of Formula (V), since use of too large an amount of solvent is uneconomical.

In the reaction of the compound of Formula (V) with a (meth)acrylic acid ester, (meth)acrylic acid, (meth)acrylic acid halide, or (meth)acrylic anhydride, it is preferable to introduce an oxygen-containing gas to the reaction system and/or add a polymerization inhibitor to the system, in order to prevent the polymerization of the product.

The polymerization inhibitor is not limited, and may be, for example, at least one member selected from the group consisting of benzoquinone and like quinone compounds; hydroquinone, hydroquinone monomethyl ether, di-t-butyl methyl phenol, and like phenolic compounds; phenylenediamine compounds, phenothiazine, and like amine compounds; N-oxyl compounds; nitroso compounds; nitrogen oxides; etc. When adding a polymerization inhibitor, the amount thereof is not limited, but is preferably about 10 to about 10,000 ppm, and more preferably about 100 to about 5,000 ppm, relative to the total amount of the compound of Formula (V) and the (meth)acrylic acid ester, (meth)acrylic acid, (meth)acrylic acid halide, and/or (meth)acrylic anhydride.

The compound of Formula (VI) is obtained in the form a solution in many cases. A highly pure compound can be obtained by concentrating the solution. Further, if necessary, distillation purification, recrystallization, extraction operation, and/or washing with water can be performed to obtain a compound with higher purity.

When the solution containing the compound of Formula (VI) is concentrated, purified by distillation, or recrystallized, it is preferable to introduce an oxygen-containing gas into the system and/or add a polymerization inhibitor to the system in order to prevent polymerization of the product.

When a catalyst is used in the production of the compound of Formula (VI), some or all of the catalyst may be neutralized or removed by adsorption using an adsorbent, before concentration, distillation purification, recrystallization, extraction operation, or washing of the solution containing the compound of Formula (VI). Usable adsorbents are not limited, and include, for example, acid clay, activated clay, alumina, silica, zeolite, ion exchange resins, acid inorganic compounds, basic inorganic compounds, etc.

<u>Step 4</u>

Step 4 is a step of reacting the compound of Formula (VI) obtained in Step 3 with water in dilute acid or in the presence of an acid compound, to obtain the monomer compound of Formula (I) of the present invention.

In Step 4, the compound of Formula (VI) may be purified before being reacted with water in a dilute acid or in the presence of an acid compound, or a solution containing the compound of Formula (VI) may be reacted, without being purified, with water in a dilute acid or in the presence of an acid compound. For purification, concentration, extraction, washing, filtration, and/or other procedures can be performed. When using a catalyst in the reaction of Step 3, some or all of the catalyst may be neutralized with an acid or removed by adsorption using an adsorbent, before reacting the compound of Formula (IV) with water in a dilute acid or in the presence of an acid compound.

The reaction of the compound of Formula (VI) with water can be performed in a dilute acid or in the presence

of an acid compound.

Examples of dilute acids include dilute hydrochloric acid, dilute hydrobromic acid, dilute nitric acid, dilute sulfuric acid, etc. Such dilute acids are not required to have high acid concentrations. Preferable dilute acids include 0.001 to 15 wt.% hydrochloric acid, 0.001 to 20 wt.% hydrobromic acid, 0.001 to 20 wt.% nitric acid; 0.001 to 20 wt.% sulfuric acid, etc. From the viewpoint of safety and handleability, 0.01 to 7 wt.% hydrochloric acid, 0.01 to 10 wt.% hydrobromic acid, 0.01 to 10 wt.% nitric acid, 0.01 to 10 wt.% sulfuric acid, etc. are more preferable. The amount of dilute acid to be used is not limited, but is preferably about 0.01 to about 10 times, and more preferably about 0.1 to about 5 times, the weight of the compound of Formula (VI). Use of dilute acid in an amount more than 10 times the weight of the compound of Formula (VI) results in low production efficiency.

Examples of acid compounds include phosphoric acid, monoalkyl phosphates, dialkyl phosphates, and like phosphoric acid compounds; paratoluene sulfonic acid, dodecylbenzenesulfonic acid, methanesulfonic acid, and like organic sulfonic acids; oxalic acid, trifluoroacetic acid, monochloroacetic acid, formic acid, acetic acid, and like carboxylic acids; metal triflates, metal tetrafluoroborates, and Lewis acids; and clay, acid clay, activated clay, silica, alumina, zeolite, cation exchange resins, and like solid acid catalysts. Preferable examples of cation exchange resins include ion exchange resins having functional groups such as sulfonic acid groups, phosphoric acid groups, carboxy groups, etc., among which ion exchange resins having sulfonic acid groups or phosphoric acid groups are more preferable. Commercially available cation exchange resins include, for example, "Amberlyst 15J WET", "Amberlyst 15 DRY", "Amberlyst 16 WET", and "Amberlyst 31 WET" (tradenames of Rohm & Haas Co.); "RCP-160M", "RCP-150H", "RCP-170H", and "PK-216" (tradenames of Mitsubishi Chemical Corp.); "Dowex 50W" (tradename of Dow Chemical Co.); etc. The amount of acid compound to be added is not limited, but when using a phosphoric acid compound, organic sulfonic acid, carboxylic acid, or Lewis acid, the amount thereof is preferably about 0.01 to about 100 mol per mol of the compound of Formula (VI), and when using a solid acid catalyst, the amount thereof is preferably about 0.1 to about 500 parts by weight per 100 parts by weight of the compound of Formula (VI). In this case, the amount of water to be present with the acid compound is not limited, and is preferably about 0.01 to about 10 times, and more preferably about 0.1 to about 5 times, the weight of the compound of Formula (VI). Use of water in an amount more than about 10 times the weight of the compound of Formula (VI) results in low production efficiency. The solid acid catalyst can be separated by filtration after the reaction. When reacting the compound of Formula (VI) with water in the presence of an acid compound, the compound of Formula (VI) may be first mixed with the acid compound, and then water may be added; or alternatively, the compound of Formula (VI) may be first mixed with water, and then the acid compound may be added.

The reaction of the compound of Formula (VI) with water can be carried out with heating and/or under pressure to increase the reaction rate, regardless of whether the reaction is carried out in a dilute acid or in the presence of an acid compound. The reaction temperature is not limited, but is usually about 0°C to the reflux temperature. Too low a reaction temperature results in a low reaction rate, whereas too high a reaction temperature may cause coloring. Therefore, reaction temperatures of about 10 to about 100°C are more preferable. The reaction can be carried out without using any solvents other than water, but may be carried out using an organic solvent in combination with water. Examples of usable organic solvents include alcohols, ester derivatives, amide derivatives, ether derivatives, hydrocarbons, and halogenated hydrocarbons, etc. The amount of solvent to be used is not limited, but is preferably not more than about 10 times the weight of the compound of Formula (VI), since use of too large an amount of solvent is uneconomical.

The reaction can also be carried out in a two-phase system. When performing the reaction in a two-phase system, an onium salt or the like may be added to increase the reaction rate. The alcohol formed in the reaction system may be removed from the system at atmospheric pressure or under reduced or elevated pressure, optionally with heating and/or while introducing a gas such as air, nitrogen, or the like. In order to prevent coloring of the product, the reaction may be carried out in an atmosphere of an inert gas such as nitrogen or the like.

When reacting the compound of Formula (VI) with water in a dilute acid or in the presence of an acid compound, it is preferable to introduce an oxygen-containing gas into the system and/or add a polymerization inhibitor to the system, in order to prevent polymerization of the product. The polymerization inhibitor is not limited, and may be, for example, at least one member selected from the above-mentioned polymerization inhibitors. When adding polymerization inhibitor (s), the amount thereof is not limited, but is preferably about 10 to about 10,000 ppm, and more preferably about 100 to about 5,000 ppm, relative to the weight of the compound of Formula (IV).

The compound represented by Formula (I) can be obtained by reacting the compound of Formula (VI) with water in a dilute acid or in the presence of an acid compound. In this case, concentration, distillation purification, recrystallization, extraction operation, and/or washing with water can be repeated as required, to obtain a compound with higher purity.

When the compound of Formula (I) is concentrated, purified by distillation, and/or recrystallized, it is preferable to introduce an oxygen-containing gas into the system and/or add a polymerization inhibitor as mentioned above to the system.

In the production of the compound of Formula (I), some or all of the dilute acid or acid compound used may be neutralized or removed by adsorption using an adsorbent, before performing concentration, distillation purification,

recrystallization, extraction operation, or washing of the solution containing the compound. Usable adsorbents are not limited, and include, for example, acid clay, activated clay, alumina, silica, zeolite, ion exchange resins, acid inorganic compounds, basic inorganic compounds, etc.

Examples of the compound of Formula (I) thus obtained include N-(2-((meth)acryloyloxy)alkyl)-2-oxoalkanamides, N-alkyl-N-(2-((meth)acryloyloxy)alkyl)-2-oxoalkanamides, etc.

Specific examples of the compound of Formula (I) include N-(2-((meth)acryloyloxy)ethyl)pyruvamide, N-(1,1-dimethyl-2-((meth)acryloyloxy)ethyl)pyruvamide, N-(1-methyl-2-((meth)acryloyloxy)ethyl)pyruvamide, N-(1-ethyl-2-((meth)acryloyloxy)ethyl)pyruvamide, N-(3-((meth)acryloyloxy)propyl)pyruvamide, N-(4-((meth)acryloyloxy)butyl)pyruvamide, N-(5-((meth)acryloyloxy)pentyl)pyruvamide, N-(6-((meth)acryloyloxy)hexyl)pyruvamide, N-(2-methyl-2-((meth)acryloyloxy)ethyl)pyruvamide, N-(2-((meth)acryloyloxy)ethyl)-N-methyl-pyruvamide, N-(1,1-dimethyl-2-((meth)acryloyloxy)ethyl)-N-methyl-pyruvamide, N-(1-methyl-2-((meth)acryloyloxy)ethyl)-N-methyl-pyruvamide, N-(1-ethyl-2-((meth)acryloyloxy)ethyl)-N-methyl-pyruvamide, N-(3-((meth)acryloyloxy)propyl)-N-methyl-pyruvamide, N-(4-((meth)acryloyloxy)butyl)-N-methyl-pyruvamide, N-(5-((meth)acryloyloxy)pentyl)-N-methyl-pyruvamide,N-(6-((meth)acryloyloxy)hexyl)-N-methyl-pyruvamide, N-(2-rriethyl-2-((meth)acryloyloxy)ethyl)-N-methyl-pyruvamide, N-(2-((meth)acryloyloxy)ethyl)-N-ethyl-pyruvamide, N-(1,1-dimethyl-2-((meth)acryloyloxy)ethyl)-N-ethyl-pyruvamide, N-(1-methyl-2-((meth)acryloyloxy)ethyl)-N-ethyl-pyruvamide, N-(1-ethyl-2-((meth)acryloyloxy)ethyl)-N-ethyl-pyruvamide, N-(3-((meth)acryloyloxy)propyl)-N-ethyl-pyruvamide, N-(4-((meth)acryloyloxy)butyl)-N-ethyl-pyruvamide, N-(5-((meth)acryloyloxy)pentyl)-N-ethyl-pyruvamide, N-(6-((meth)acryloyloxy)hexyl)-N-ethyl-pyruvamide, N-(2-methyl-2-((meth)acryloyloxy)ethyl)-N-ethyl-pyruvamide, N-(2-((meth)acryloyloxy)ethyl)-N-isopropyl-pyruvamide, N-(1,1-dimethyl-2-((meth)acryloyloxy)ethyl)-N-isopropyl-pyruvamide, N-(1-methyl-2-((meth)acryloyloxy)ethyl)-N-isopropyl-pyruvamide, N-(1-ethyl-2-((meth)acryloyloxy)ethyl)-N-isopropyl-pyruvamide, N-(3-((meth)acryloyloxy)propyl)-N-isopropyl-pyruvamide, N-(4-((meth)acryloyloxy)butyl)-N-isopropyl-pyruvamide, N-(5-((meth)acryloyloxy)pentyl)-N-isopropyl-pyruvamide, N-(6-((meth)acryloyloxy)hexyl)-N-isopropyl-pyruvamide, N-(2-methyl-2-((meth)acryloyloxy)ethyl)-N-isopropyl-pyruvamide, N-(2-((meth)acryloyloxy)ethyl)-N-butyl-pyruvamide, N-(1,1-dimethyl-2-((meth)acryloyloxy)ethyl)-N-butyl-pyruvamide, N-(1-methyl-2-((meth)acryloyloxy)ethyl)-N-butyl-pyruvamide, N-(1-ethyl-2-((meth)acryloyloxy)ethyl)-N-butyl-pyruvamide, N-(3-((meth)acryloyloxy)propyl)-N-butyl-pyruvamide, N-(4-((meth)acryloyloxy)butyl)-N-butyl-pyruvamide, N-(5-((meth)acryloyloxy)pentyl)-N-butyl-pyruvamide, N-(6-((meth)acryloyloxy)hexyl)-N-butyl-pyruvamide, N-(2-methyl-2-((meth)acryloyloxy)ethyl)-N-butyl-pyruvamide, N-(2-((meth)acryloyloxy)ethyl)-N-t-butyl-pyruvamide, N-(1,1-dimethyl-2-((meth)acryloyloxy)ethyl)-N-t-butyl-pyruvamide, N-(1-methyl-2-((meth)acryloyloxy)ethyl)-N-t-butyl-pyruvamide, N-(1-ethyl-2-((meth)acryloyloxy)ethyl)-N-t-butyl-pyruvamide, N-(3-((meth)acryloyloxy)propyl)-N-t-butyl-pyruvamide, N-(4-((meth)acryloyloxy)butyl)-N-t-butyl-pyruvamide, N-(5-((meth)acryloyloxy)pentyl)-N-t-butyl-pyruvamide, N-(6-((meth)acryloyloxy)hexyl)-N-t-butyl-pyruvamide, N-(2-methyl-2-((meth)acryloyloxy)ethyl)-N-t-butyl-pyruvamide, etc.

The compound of Formula (I) is preferably a compound in which Y is an ethylene group which may have a $C_{1-6}$ organic group as a substituent; a compound in which Y is a straight-chain propylene group which may have a $C_{1-6}$ organic group as a substituent; or the like.

More preferably, from the viewpoint of availability of starting materials, the compound of Formula (I) is a compound in which Y is an ethylene or a straight-chain propylene group, or the like. Examples of such compounds include N-(2-((meth)acryloyloxy)ethyl)pyruvamide, N-(1,1-dimethyl-2-((meth)acryloyloxy)ethyl)pyruvamide, N-(1-ethyl-2-((meth)acryloyloxy)ethyl)pyruvamide, N-(3-((meth)acryloyloxy)propyl)pyruvamide, N-(2-((meth)acryloyloxy)ethyl)-N-methyl-pyruvamide, N-(2-((meth)acryloyloxy)ethyl)-N-ethyl-pyruvamide, N-(2-((meth)acryloyloxy)ethyl)-N-isopropyl-pyruvamide, N-(2-((meth)acryloyloxy)ethyl)-N-butyl-pyruvamide, N-(2-((meth)acryloyloxy)ethyl)-N-t-butyl-pyruvamide, etc.

Also preferable as the compound of Formula (I) is a compound in which Y is an ethylene group, $R^1$ is a methyl group, $R^2$ is a hydrogen atom or a $C_{1-4}$ alkyl group, in particular a methyl group, and $R^3$ is a hydrogen atom or a methyl group, since starting materials for such compounds are inexpensive. Examples of such compounds include N-(2((meth)acryloyloxy)ethyl)pyruvamide, N-(2-((meth)acryloyloxy)ethyl)-N-methyl-pyruvamide, etc.

<u>Polymer of monomer compound of Formula (I)</u>

Since the monomer compound of Formula (I) contains a (meth)acryloyloxy group, it can be homopolymerized, and can also be copolymerized with other vinyl monomer(s).

Polymers obtained by homopolymerizing the monomer compound or copolymerizing the compound with other vinyl monomer(s) have $\alpha$-oxoamide groups represented by Formula (VII)

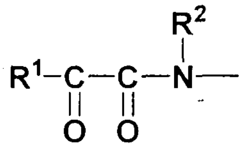 (VII)

wherein $R^1$ and $R^2$ are as defined above. The α-oxoamide groups react with a crosslinking agent that is reactive with carbonyl groups, and is thereby cured. Therefore, the polymers are useful as base resins for various curable compositions.

It is usually preferable that such polymers contain α-oxoamide groups of Formula (VII) at a molar concentration of about 0.05 to about 5.5 mol/kg, and more preferably about 0.1 to about 5 mol/kg.

The number average molecular weight of polymers obtained by homopolymerizing the monomer compound of Formula (I) or copolymerizing the compound with other vinyl monomer(s) is not limited, but is usually about 1,000 to about 2,000,000.

Examples of other vinyl monomers are not limited, and any known vinyl monomers are usable. The following are examples of usable other vinyl monomers:

(1) $C_{1-30}$ alkyl esters or cycloalkyl esters of acrylic acid and methacrylic acid, such as methyl (meth)acrylate, ethyl (meth)acrylate, n-propyl (meth)acrylate, isopropyl (meth)acrylate, n-butyl (meth)acrylate, i-butyl (meth)acrylate, t-butyl (meth)acrylate, hexyl (meth)acrylate, 2-ethylhexyl (meth)acrylate, n-octyl (meth)acrylate, decyl (meth)acrylate, lauryl (meth)acrylate, stearyl (meth)acrylate, cyclohexyl (meth)acrylate, isobornyl (meth)acrylate, etc.;
(2) $C_{2-18}$ alkoxyalkyl esters of acrylic acid and methacrylic acid, such as methoxybutyl (meth)acrylate, methoxyethyl (meth)acrylate, ethoxybutyl (meth)acrylate, etc.; and acetoacetoxylethyl (meth)acrylate;
(3) vinyl aromatic compounds, such as styrene, α-methylstyrene, vinyltoluene, p-chlorostyrene, etc.;
(4) $C_{2-8}$ hydroxyalkyl esters of acrylic acid and methacrylic acid, such as 2-hydroxyethyl (meth)acrylate, hydroxypropyl (meth)acrylate, hydroxybutyl (meth)acrylate, etc.
(5) adducts of hydroxyalkyl esters of acrylic acid and methacrylic acid with ε-caprolactone, such adducts being commercially available as, for example, "Placcel FM-3" (tradename of Daicel Chemical Industries, Ltd.);
(6) polyalkylene glycol mono(meth)acrylates and hydroxyalkyl vinyl ethers;
(7) epoxy group-containing unsaturated monomers, such as glycidyl (meth)acrylate, diglycidyl fumarate, allyl glycidyl ether, ε-caprolactone-modified glycidyl (meth)acrylate, β-methylglycidyl (meth)acrylate, 3,4-epoxycyclohexylmethyl (meth)acrylate, etc.;
(8) perfluoroalkyl esters of acrylic acid and methacrylic acid, such as perfluorobutylethyl (meth)acrylate, perfluoroisononylethyl (meth)acrylate, perfluorooctylethyl (meth)acrylate, etc.;
(9) olefins, such as ethylene, propylene, butylene, pentene, etc.;
(10) diene compounds, such as butadiene, isoprene, chloroprene, etc.;
(11) fluoroolefins, such as trifluoroethylene, tetrafluoroethylene, vinylidene fluoride, etc.;
(12) vinyl esters and propenyl esters of $C_{1-20}$ fatty acids, such as vinyl acetate, vinyl propionate, vinyl caprate, isopropenyl acetate, etc.; and vinyl ethers, such as ethyl vinyl ether, n-propyl vinyl ether, isopropyl vinyl ether, butyl vinyl ether, octyl vinyl ether, cyclohexyl vinyl ether, phenyl vinyl ether, benzyl vinyl ether, etc., such vinyl ethers being commercially available as, for example, "Veova monomer" (tradename of Shell Chemical Co.; vinyl ester of a branched-chain higher fatty acid) and the like;
(13) carboxy-containing unsaturated monomers, such as acrylic acid, methacrylic acid, crotonic acid, itaconic acid, maleic acid, fumaric acid, 2-carboxyethyl (meth)acrylate, 2-carboxypropyl (meth)acrylate, 5-carboxypentyl (meth) acrylate, etc.;
(14) hydrolyzable alkoxysilyl group-containing unsaturated monomers, such as vinyltriethoxysilane, vinyltrimethoxysilane, vinyltris(methoxyethoxy)silane, γ-methacryloyloxypropyltrimethoxysilane, 2-styrylethyltrimethoxysilane, etc.;
(15) nitrogen-containing alkyl (meth)acrylates, such as N,N-dimethylaminoethyl (meth)acrylate, N-t-butylaminoethyl (meth)acrylate, etc.;
(16) polymerizable amides, such as acrylamide, methacrylamide, N-methyl (meth)acrylamide, N-ethyl (meth)acrylamide, N-methylol (meth)acrylamide, N-methoxymethyl (meth)acrylamide, N-butoxymethyl (meth)acrylamide, N,N-dimethyl (meth)acrylamide, N,N-dimethylaminopropyl (meth)acrylamide, N,N-dimethylaminoethyl (meth)acrylamide, diacetone acrylamide, etc.; and nitrogen-containing monomers, such as 2-vinylpyridine, 1-vinyl-2-pyrrolidone, 4-vinylpyridine, (meth)acryloylmorpholine, etc.;
(17) sulfonic acid monomers, such as vinylsulfonic acid, methallylsulfonic acid, sulfoethyl (meth)acrylate, styrenesulfonic acid, 2-acrylamide-2-methylpropanesulfonic acid, etc., and salts thereof;

(18) esters of hydroxy-containing monomers with phosphoric acid compounds, such as 2-(meth)acryloyloxyethyl acid phosphate;

(19) phosphoric acid group-containing monomers, such as products obtained by adding phosphoric acid compounds to epoxy groups of glycidyl (meth)acrylate or the like;

(20) polymerizable nitriles, such as acrylonitrile, methacrylonitrile, etc.;

(21) polymerizable amines,such as allylamine and the like; and

(22) acid anhydride monomers, such as maleic anhydride, itaconic anhydride, etc.

Known polymerization methods can be used, including, for example, radical polymerization, coordination polymerization, group transfer polymerization (functional group transfer polymerization), ATRP (atom transfer polymerization), chain transfer polymerization, etc.

Known polymerization forms are usable, including, for example, homogeneous polymerization in a solution, batch polymerization, emulsion polymerization, dispersion polymerization, suspension polymerization, etc.

It is particularly preferable to polymerize or copolymerize the monomer compound of Formula (I) in an organic solvent or in an emulsified state in water.

For example, the polymer of the present invention can be synthesized by copolymerizing the monomer compound of Formula

(I) with other vinyl monomer(s) in an organic solvent using a polymerization initiator.

Organic solvents that can be used for such solution polymerization include, for example, toluene, xylene, "Swasol 1000" (tradename of COSMO OIL CO., LTD.; petroleum solvent with a high boiling point), and like aromatic solvents; ethyl acetate, 3-methoxybutyl acetate, ethylene glycol ethyl ether acetate, propylene glycol methyl ether acetate, and like ester solvents; methyl ethyl ketone, methyl isobutyl ketone, methyl amyl ketone, and like ketone solvents; ethylene glycol monobutyl ether, propylene glycol monopropyl ether, butanol, and like alcohol solvents; propyl propionate, butyl propionate, and ethoxy ethyl propionate; etc. Such organic solvents can be used singly or in combination.

Usable polymerization initiators include, for example, 2,2'-azobisisobutyronitrile, benzoyl peroxide, di-t-butyl peroxide, di-t-amyl peroxide, t-butyl peroctoate, 2,2'-azobis(2-methylbutyronitrile), and like known radical polymerization initiators. The amount of polymerization initiator to be used is preferably about 0.01 to 30 wt.%, and more preferably about 0.1 to 20 wt.%, relative to the weight of monomers to be polymerized.

The polymerization conditions are usually such that the reaction temperature is about room temperature to about 200°C, and the reaction time is about 2 to about 10 hours.

Further, the polymer of the present invention can be synthesized by, for example, copolymerizing the monomer compound of Formula (I) and other vinyl monomer(s) in an emulsified state in water, in the presence of a dispersion stabilizer such as a surfactant, using a polymerization initiator.

Examples of usable dispersion stabilizers include anionic emulsifiers, nonionic emulsifiers, amphoteric emulsifiers, etc. Specific examples of anionic emulsifiers include fatty acids, alkyl sulfonate salts, alkylbenzene sulfonic acid salts, alkyl phosphoric acid salts, etc. Specific examples of nonionic emulsifiers include polyoxyethylene alkyl ethers, polyoxyethylene alkyl allyl ethers, polyoxyethylene derivatives, sorbitan fatty acid esters, polyoxyethylene sorbitan fatty acid esters, polyoxyethylene alkylamines, alkyl alkanolamides, etc. Specific examples of amphoteric emulsifiers include alkyl betaines and the like. The concentration of emulsifier is usually 0.1 to 10 wt.%, and preferably 1 to 5 wt.%, relative to the weight of monomer(s) to be polymerized.

Usable polymerization initiators include, for example, ammonium persulfate, 4,4'-azobis(4-cyanobutanoic acid), and like radical polymerization initiators. The amount of polymerization initiator is usually about 0.01 to about 10 wt.%, and preferably about 0.1 to about 5 wt.%, relative-to the weight of monomer(s) to be polymerized.

The polymerization conditions are usually such that the reaction temperature is about 60 to about 90°C, and the reaction time is about 5 to about 10 hours.

Among the polymers of the present invention, those having at least one anionic group selected from carboxylate groups, phosphate groups, and sulfonate groups, as well as $\alpha$-oxoamide groups of Formula (VII), can be advantageously used for aqueous curable compositions. Polymers having such anionic groups can be prepared by, for example, the following method (1) or (2).

(1) Copolymerizing the monomer compound of Formula (I) of the present invention with other vinyl monomer(s) including at least one monomer selected from carboxy-containing vinyl monomers, phosphoric acid group-containing vinyl monomers, and sulfonic acid group-containing monomers; followed by neutralizing some or all of the carboxy groups, phosphoric acid groups, and sulfonic acid groups, with a basic compound.

(2) Copolymerizing the monomer compound of Formula (I) of the present invention with other monomer(s) including at least one anionic group-containing vinyl monomer selected from carboxylate group-containing vinyl monomers,

phosphate group-containing vinyl monomers, and sulfonate group-containing vinyl monomers.

Examples of carboxy-containing vinyl monomers include acrylic acid, methacrylic acid, crotonic acid, itaconic acid, maleic acid, fumaric acid, 2-carboxyethyl (meth)acrylate, 2-carboxypropyl (meth)acrylate, 5-carboxypentyl (meth) acrylate, and like carboxy-containing unsaturated monomers.
Examples of carboxylate group-containing vinyl monomers include salts of the above-mentioned carboxy-containing vinyl monomers.
Examples of phosphoric acid group-containing vinyl monomers include esters of phosphoric acid compounds with hydroxy-containing unsaturated monomers such as 2-(meth)acryloyloxyethyl acid phosphate.
Examples of phosphate group-containing vinyl monomers include salts of the above-mentioned phosphoric acid group-containing vinyl monomers.
Examples of sulfonic acid group-containing vinyl monomers include vinylsulfonic acid, methallylsulfonic acid, sulfoethyl (meth)acrylate, styrenesulfonic acid, 2-acrylamide-2-methylpropanesulfonic acid, and like sulfonic acid monomers.
Examples of sulfonate group-containing vinyl monomers include salts of the above-mentioned sulfonic acid group-containing vinyl monomers.
Among the polymers of the present invention, those containing, in addition to the $\alpha$-oxoamide groups of Formula (VII), at least one nonionic hydrophilic group selected from polyoxyethylene groups, polyoxypropylene groups, and polyoxyethylene polyoxypropylene groups, can also be advantageously used for aqueous curable compositions. Such nonionic hydrophilic group-containing polymers can be prepared by, for example, the following method.
Copolymerizing the monomer compound of Formula (I) of the present invention with other vinyl monomer(s) including at least one nonionic hydrophilic group-containing vinyl monomer selected from monoalkyl polyoxyethylene group-containing vinyl monomers, monoalkyl polyoxypropylene group-containing vinyl monomers, monoalkyl polyoxyethylene polyoxypropylene group-containing vinyl monomers, hydroxy(polyoxyethylene) group-containing vinyl monomers, hydroxy(polyoxypropylene) group-containing vinyl monomers, and hydroxy(polyoxyethylene polyoxypropylene) group-containing vinyl monomers.

Aqueous curable composition

The aqueous curable composition of the present invention comprises (A) a polymer of the monomer compound of Formula (I), and (B) a compound having, per molecule, at least two crosslinkable groups selected from the group consisting of primary amino groups, secondary amino groups, and ammonium groups.

Compound (B)

The compound (B) is a compound having, per molecule, at least two crosslinkable groups of at least one type selected from the group consisting of primary amino groups, secondary amino groups, and ammonium groups. Preferable ammonium groups include primary ammonium groups and secondary ammonium groups.
The crosslinkable groups are reactive with $\alpha$-oxoamide group(s) of Formula (VII) in the polymer (A). Therefore, the compound (B) functions as a crosslinking agent for the polymer (A).
Some or all of primary amino groups and/or secondary amino groups in the compound (B) may be neutralized with a Brönsted acid.
Brönsted acids that can be used for neutralization include hydrochloric acid, sulfuric acid, phosphoric acid, and other inorganic acids; formic acid, oxalic acid, acetic acid, propionic acid, butyric acid, and like low-molecular-weight organic carboxylic acids; oxyacetic acid, lactic acid, and like oxyacids; etc.
Examples of compounds having, per molecule, at least two primary or secondary amino groups are not limited, and include (polyamino)alkanes, (polyamino)polyethers, poly(ethyleneimine)s, poly(aminoalkene)s, etc.
Specific examples of such compounds include about $C_2$ to about $C_{20}$ polymethylenediamines and such polymethylenediamines having an organic substituent at the N-position, such as ethylenediamine, N,N'-dimethylethylenediamine, propylenediamine, trimethylenediamine, tetramethylenediamine, pentamethylenediamine, hexamethylenediamine, heptamethylenediamine, etc.; oxyalkylene group- or polyoxyalkylene group-containing diamines, such as ethylene glycol bis(2-aminoethyl)ether, diethylene glycol bis(2-aminoethyl)ether, tripropylene glycol (2-aminoethyl)ether, etc.; about $C_3$ to about $C_{10}$ alicyclic diamines, such as 1,2-cyclohexyldiamine, 1,4-cyclohexyldiamine, etc.; about $C_6$ to about $C_{20}$ aromatic diamines, such as o-xylylenediamine, m-xylylenediamine, p-xylylenediamine, 1,2-phenylenediamine, 1,3-phenylenediamine, 1,4-phenylenediamine, 1,4-naphthylenediamine, 4,4'-diaminodiphenylmethane, 4,4'-diaminodiphenylsulfone, etc.; about $C_4$ to about $C_{20}$ polyamines and such polyamines having an organic substituent at the N-position, such as diethylenetriamine, N-ethyldiethylenetriamine, triethylenetetramine, tetraethylenepentamine, pentaethylenehexamine, and like polyethylene polyamines, etc.; about $C_3$ to about $C_{20}$ aliphatic triamines, such as 1,2,3-triaminopropane and the like; about $C_6$ to about $C_{20}$ aromatic triamines, such as 1,2,3-aminobenzene and the like; polyoxyalkylene group-containing polyamines having such a structure that at least two hydroxy groups of a compound obtained by reacting a polyol such as trimethylolpropane, pentaerythritol, or the like, with alkylene oxide(s) such as ethylene oxide and/or propylene oxide are converted to amino groups; polyamines obtained by further reacting the above-mentioned diamines, triamines, or polyamines, with alkylene oxide(s) such as ethylene oxide and/or propylene oxide; and the like.

Cationic resins can also be used as the compound (B). Usable cationic resins are not limited, and include, for example, resins produced by reacting epoxy group-containing compounds or resins derived from epoxy group-containing compounds, with primary and/or secondary organic amines or derivatives thereof; copolymers of amino group-containing polymerizable unsaturated compounds with vinyl monomers; amino group-containing polymerizable unsaturated compounds, amide group-containing polymerizable unsaturated compounds, and copolymers of vinyl monomers; polyamide resins; cationic resins that become water-dispersible when acid compounds are added thereto; resins obtained by reacting epoxy group-containing compounds or resins derived from epoxy group-containing compounds, with cationizing agents; resins obtained by protonating, with acids, polycondensates of polycarboxylic acids with polyamines; resins obtained by protonating, with acids, polyadducts of polyisocyanate compounds, polyols, and mono- or polyamines; resins obtained by protonating, with acids, adducts of polycarboxylic acid resins with alkyleneimines; etc.

The above-mentioned cationic resins may be modified with vinyl copolymers, polybutadienes, unsaturated group-containing alkyd resins, polyesters, polyurethanes, polycarbonates, polyols, polyamines, polycarboxylic acids, ε-caprolactone, derivatives thereof, etc. Such cationic resins may be further reacted with polyisocyanates when being modified.

Preferable epoxy group-containing compounds and resins derived from epoxy group-containing compounds include those produced by using active hydrogen-containing compounds and epichlorohydrin as starting materials. Examples of active hydrogen-containing compounds include polyphenol compounds, polyether polyols, polyester polyols, polyamide amine, polycarboxylic acids, etc. Such epoxy group-containing compounds and resins derived from epoxy group-containing compounds preferably have a number-average-molecular weight of at least about 200, more preferably about 400 to about 4,000, and even more preferably about 800 to about 2,000.

Polyphenol compounds, which are active hydrogen-containing compounds, include, for example, 2,2-bis(4-hydroxyphenyl)propane, 4,4-dihydroxybenzophenone, 1,1-bis(4-hydroxyphenyl)ethane, 1,1-bis(4-hydroxyphenyl)isobutane, 2,2-bis(4-hydroxy-tert-butyl-phenyl)propane, bis(2-hydroxynaphthyl)methane, 1,5-dihydroxynaphthalene, bis (2,4-dihydroxyphenyl)methane, tetra-1,1,2,2-(4-hydroxyphenyl)ethane, 4,4-dihydroxydiphenylsulfone, phenol novolac, cresol novolac, etc.

Examples of usable cationic resins include the following (i) to (iii):

(i) adducts of epoxy group-containing compounds or resins derived from epoxy group-containing compounds, with monoamines or polyamines, as described in, for example, U.S. Pat. No. 3,984,299 specification;

(ii) adducts of epoxy group-containing compounds or resins derived from epoxy group-containing compounds, with secondary monoamines having ketiminized primary amino groups, and polyamines, as described in, for example, U.S. Pat. No. 4,017,438 specification.

(iii) reaction products obtained by etherification of epoxy group-containing compounds or resins derived from epoxy group-containing compounds, with hydroxy compounds having ketiminized primary amino groups, as described in Japanese Unexamined Patent Application Publication No. 1984-43013.

Preferable cationic resins for use as the compound (B) include those produced by using as starting materials an amine compound and a compound having at least two glycidyl groups per molecule. The amine compound may be a ketiminized amine compound.

Examples of compounds having at least two glycidyl groups per molecule include diglycidyl ether-terminated aromatic ring-containing resins derived from bisphenol A, such as "Epikote 828", "Epikote 1001", "Epikote 1002" (all tradenames of Japan Epoxy Resin Co., Ltd.), etc.; diglycidyl ether-type resins derived from bisphenol F, such as "Epikote 806", "Epikote 4004P", "Epikote 4007P" (all tradenames of Japan Epoxy Resin, Co., Ltd.), etc.; polyglycidyl ether-terminated aliphatic resins, such as ethylene glycol diglycidyl ether, neopentyl glycol diglycidyl ether, glycerol polyglycidyl ether, trimethylolpropane triglycidyl ether, polyethylene glycol diglycidyl ether, polypropylene glycol diglycidyl ether, etc.; and the like.

Examples of amine compounds include monoamines, diamines, triamines, polyamines, etc. Monoamines include, for example, butylamine, dibutylamine, 2-ethylhexylamine, octylamine, dodecylamine, stearylamine, and like $C_{4-18}$ aminoalkanes; ethanolamine, 2- (N-methylamino) ethanol, (2-hydroxyethoxy)ethylamine, 2-amino-2-methyl-l-propanol, and like oxygen atom-containing monamino compounds; etc. Usable diamines, triamines, and polyamines include those mentioned above.

Examples of ketiminized amine compounds include ketimines obtained by reacting the primary amino group

of diethylenetriamine with ketones such as methyl isobutyl ketone or the like; and compounds obtained by reacting the amino group of 2-aminoethanol with ketones, and like amine derivatives in which some or all of the amino groups are ketiminized. When a ketiminized amine compound is reacted with an amine compound and/or a compound having at least two glycidyl groups per molecule, it is possible to hydrolyze the ketimine moiety and thereby regenerate amino groups by adding water and, if necessary, a Brönsted acid such as acetic acid, formic acid, or the like.

The compound (B) may have cationic groups such as ammonium groups, sulfonium groups, phosphonium groups, etc.

Such a cationic group-containing compound (B) can be synthesized by, for example, further reacting epoxy groups of an epoxy group-containing cationic resin as mentioned above, with a tertiary amine salt, secondary sulfide salt, or tertiary phosphine salt.

Examples of tertiary amine salts include salts of tertiary amines such as triethylamine, triethanolamine, N,N-dimethylethanolamine, N-methyldiethanolamine, N,N-diethylethanolamine, N-ethyldiethanolamine, etc., with inorganic acids such as hydrochloric acid, sulfuric acid, phosphoric acid, etc.; low molecular-weight organic carboxylic acids such as oxalic acid, acetic acid, formic acid, propionic acid, etc.; or oxyacids such as oxyacetic acid, lactic acid, etc.

Examples of secondary sulfide salts include salts of sulfides such as diethylsulfide, diphenylsulfide, tetramethylenesulfide, thiodiethanol, etc., with boric acid, carbonic acid, organic monocarboxylic acid, etc.

Examples of tertiary phosphine salts include salts of phosphines such as triethylphosphine, phenyldimethylphosphine, diphenylmethylphosphine, triphenylphosphine, with acids as mentioned above.

The compound (B) may further contain nonionic hydrophilic group(s). A nonionic hydrophilic group-containing compound (B) can be obtained, for example, by copolymerizing an amino group-containing acrylic monomer or amino group-containing vinyl monomer, a nonionic hydrophilic group-containing vinyl monomer, and other vinyl monomers. Examples of nonionic hydrophilic group-containing vinyl monomers include monoalkylpolyoxyethylene group-containing vinyl monomers, monoalkylpolyoxypropylene group-containing vinyl monomers, monoalkylpolyoxyethylene polyoxypropylene group-containing vinyl monomers, hydroxy(polyoxyethylene) group-containing vinyl monomers, hydroxy(polyoxypropylene) group-containing vinyl monomers, hydroxy(polyoxyethylenepolyoxypropylene) group-containing vinyl monomers, etc. Other usable vinyl monomers include monomers (1) to (22) mentioned above as monomers to be copolymerized with the compound of Formula (I).

The compound (B) may be a compound obtained by reacting a polyalkylene glycol compound, an epoxy group-containing compound and/or resin derived from an epoxy group-containing compound, and a polyisocyanate. The compound (B) may also be a compound obtained by reacting a polyalkylene glycol compound, a polyamine resin as mentioned above, and a polyisocyanate. The compound (B) may also be a compound obtained by reacting a polyalkylene glycol compound, an epoxy group-containing compound and/or resin derived from an epoxy group-containing compound, a polyamine resin, and a polyisocyanate. Such compounds (B) can also be obtained by further reacting monoamines, diamines, triamines, polyamines or like amine compounds. Examples of polyalkylene glycol compounds include polyethylene glycols, polyethylene glycol monoalkyl ethers, and like hydroxy-containing polyoxyethylene derivatives; polypropylene glycols, polypropylene glycol monoalkyl ethers, and like hydroxy-containing polyoxypropylene derivatives; polyethylene polypropylene glycols, polyethylene polypropylene glycol monoalkyl ethers, and like hydroxy-containing polyoxyethylene polyoxypropylene derivatives; etc.

In the aqueous curable composition of the present invention, the molar concentration of $\alpha$-oxoamide groups of Formula (VII) in the polymer (A) is preferably about 0.05 to about 5.5 mol/kg, and more preferably about 0.1 to about 5 mol/kg. The molar concentration of crosslinkable groups in the compound (B) is preferably about 0.05 to about 35 mol/kg, and more preferably about 0.1 to about 25 mol/kg. If the molar concentration of $\alpha$-oxoamide groups in the polymer (A) or the molar concentration of crosslinkable groups in the compound (B) is excessively lower than the above ranges, it becomes difficult to cure the composition, whereas if it is excessively higher than the above ranges, the production cost for the composition becomes high. Thus, excessively low and high molar concentrations are not preferable.

The ratio of the polymer (A) to the compound (B) is preferably such that the ratio of the $\alpha$-oxoamide groups of Formula (VII) in the polymer (A) to the crosslinkable groups in the compound (B) is (the number of moles of the $\alpha$-oxoamide groups)/(the number of moles of the crosslinkable groups) = about 0.3 to about 3, and more preferably about 0.5 to about 2. When the ratio of the number of moles of the $\alpha$-oxoamide groups to the number of moles of the crosslinkable groups is less than 0.3 or more than 3, the composition may not be cured. Thus, such ratios are not preferable.

When preparing an anionic aqueous curable composition, in order to improve the water dispersion stability, the polymer (A) preferably has carboxy group(s) and/or carboxylate group(s). To improve the thermal load stability with the passage of time during storage of the composition, it is preferable to prepare an aqueous curable composition by mixing the polymer (A) with the amine compound (B) so that the ratio of carboxy and carboxylate groups in the polymer (A) to crosslinkable groups in the compound (B) becomes (the total number of moles of the carboxy and carboxylate groups)/(the number of moles of the crosslinkable groups) = about 0.05 to about 10, and more preferably about 0.1 to about 7.

In order to improve the water resistance of coating films obtained from the curable composition, the total molar

concentration of carboxy and carboxylate groups in the polymer (A) in the composition is preferably about 0.05 to about 2 mol/kg, and more preferably about 0.07 to about 1.4 mol/kg.

When preparing a cationic aqueous curable composition, from the viewpoint of water dispersion stability, it is preferable that the compound (B) contain ammonium group(s). The molar concentration of ammonium groups is preferably about 0.1 to about 6 mol/kg, and more preferably about 0.2 to about 5 mol/kg. When the molar concentration is less than 0.1 mol/kg, water dispersion stability may deteriorate, whereas when it is more than 6 mol/kg, water resistance may become poor.

The aqueous curable composition of the present invention is obtained by dispersing the polymer (A) and the compound (B) in water or an aqueous medium consisting of water and organic solvent. Examples of organic solvents include ethanol, isopropanol, propylene glycol monopropyl ether, diethylene glycol monomethyl ether, and like alcohols; ethyl acetate and like esters; acetone, methyl ethyl ketone, and like ketones; diprbpylene glycol dimethyl ether and like ethers; hexane, heptane, and like aliphatic hydrocarbons; toluene, xylene, and like aromatic hydrocarbons; etc.

The aqueous curable composition has high crosslinking efficiency, since, when it is formed into a coating film, the dehydration condensation reaction of $\alpha$-oxoamide groups in the polymer (A) with crosslinkable groups in the compound (B) proceeds smoothly due to the evaporation of the aqueous medium. Therefore, the crosslinking reaction proceeds even at room temperature, thereby forming a crosslinked coating film having good resistance to heat, solvents and water. If necessary, the curable aqueous composition of the present invention may be heated to promote crosslinking.

The aqueous curable composition of the present invention can be prepared by a simple procedure, i.e., mixing the polymer (A) with the compound (B) by a known method such as stirring, shaking, etc. The polymer (A) and the compound (B) may be solutions or dispersions in aqueous media as mentioned above, and if necessary, may be diluted with such aqueous media.

The aqueous curable composition of the present invention may contain various additives such as film formation auxiliaries, surfactants and like dispersants, emulsifiers, surface controlling agents, preservatives, antimicrobial agents, antifoaming agents, coloring pigments, extender pigments, fillers, thickeners, plasticizers, antirust agents, etc., according to the use of the composition.

The aqueous curable composition of the present invention finds a wide range of applications such as aqueous coating compositions, aqueous bonding agents, aqueous tacky adhesives, aqueous paper processing agents, aqueous inks, aqueous hair spray compositions, aqueous cosmetics, aqueous hair dyes, aqueous manicure preparations, aqueous photosensitive resin compositions, aqueous polymer modifiers, aqueous ion exchange resin compositions, aqueous carrier resin compositions, aqueous resin compositions for controlled release of drugs, etc.

The aqueous curable composition of the present invention can be applied by known methods such as air spraying, airless spraying, electrostatic coating, brush coating, electrodeposition coating, etc. The applied coating preferably has a thickness of 3 to 100 $\mu$m, and more preferably 5 to 60 $\mu$m, when cured.

The aqueous curable composition of the present invention can be applied by electrodeposition coating to a substrate with an electrically conductive metal surface. Specific examples of substrates include automobile bodies, electrical products, etc.

In such cases, when the composition of the present invention is a cationic composition, it is applied by the known method in which electrodeposition is performed using the substrate as the cathode and providing an anode. When the composition of the present invention is an anionic composition, it can be applied by using a cathode and anode in a reverse manner to the case of cationic compositions.

More specifically, for example, a cationic composition, can be applied by electrodeposition using the substrate as the cathode and a carbon plate as the anode, at a bath temperature of about 20 to about 35°C, a voltage of about 100 to about 400 V, a current density of about 0.01 to about 5 A, and a current application time of about 1 to about 10 minute. The resulting coating film preferably has a thickness of about 10 to about 40 $\mu$m, and more preferably about 15 to about 30 $\mu$m, when cured by heating.

EFFECTS OF THE INVENTION

The present invention achieves the following remarkable effects.

(1) The monomer compound of Formula (I) of the present invention can be produced safely, since the production thereof does not require acrylonitrile, which has high toxicity and low boiling point, or concentrated sulfuric acid, which is strongly acidic and highly corrosive, as starting materials.

(2) The monomer compound of Formula (I) has $\alpha$-oxoamide and (meth)acryloyloxy groups, and polymers of the monomer compound are not decomposed by reverse Claisen condensation, since they have an $\alpha$-diketo-type structure and do not have $\beta$-diketo-type acetoacetoxy groups.
Therefore, polymers obtained by homopolymerizing or copolymerizing the monomer compound have excellent storage stability.

(3) Even when the monomer compound of Formula (I) is polymerized in an aqueous system, the obtained polymer does not undergo decomposition or the like. The obtained polymer is extremely stable when used for producing a coating composition, and the α-oxoamide groups in the polymer have excellent reactivity with the compound (B) used as a crosslinking agent.

(4) Since homopolymers and copolymers of the monomer compound of Formula (I) have excellent storage stability, the aqueous curable composition of the present invention, which comprises a homopolymer or copolymer of the monomer compound and the compound (B), has extremely high storage stability and excellent room temperature curability.

BEST MODE FOR CARRYING OUT THE INVENTION

The following Production Examples, Examples, and Comparative Examples are provided to illustrate the present invention in further detail, and are not intended to limit the scope of the invention. Various modifications may be made within the scope of the invention.

In these examples, the product purity (%), heating residue (%), polymerization conversion of monomer (%), and carbonyl decomposition rate (%) were determined by the following methods.

Product purity (%): The product purity was calculated from the peak area of gas chromatography, according to the following formula.

$$\text{Purity (\%)} = (A/B) \times 100$$

wherein A is the peak area of the desired product, and B is the total peak area.

Heating residue (%): A sample solution or dispersion was placed on a tin dish, and dried by heating in a drying furnace at 125°C for 3 hours. The remainder was weighed, and the heating residue was calculated according to the following formula.

$$\text{Heating residue (\%)} = (C/D) \times 100$$

wherein C is the weight of the remainder, and D is the weight of the sample solution or dispersion.

Polymerization conversion of monomer (%): The conversion was calculated according to the following formula.

$$\text{Conversion (\%)} = \text{heating residue (\%)}/[(\text{total weight of monomers} + \text{weight of polymerization initiator})/(\text{weight of solvent} + \text{total weight of monomers} + \text{weight of polymerization initiator} + \text{weight of additives})]$$

wherein additives mean components other than monomers and polymerization initiator.

Carbonyl decomposition rate (%): The rate was calculated as follows.

(1) When the carbonyl groups were α-oxoamide groups:

$$\text{Decomposition rate (\%)} = [1-\{(S_1/S_0)/(A_1/A_0)\}] \times 100$$

wherein $S_1/S_0$ is the ratio of the area $S_1$ of the peak (62.4 (broad)) attributed to N-(2-(methacryloyloxy)ethyl)-N-methyl-pyruvamide to the area $S_0$ of the peak attributed to styrene (86.6-7.3 (broad)), the peaks being obtained by nuclear magnetic resonance spectroscopic analysis ($^1$H-NMR analysis ($CDCl_3$)) of a powder obtained by drying a resin dispersion under vacuum.

$A_1/A_0$ is as follows.

$$A_1 = (W_1/213) \times 3$$

$$A_0 = (W_0/104) \times 5$$

wherein $W_1$ and $W_0$ are the weights of N-(2-(methacryloyloxy)ethyl)-N-methyl-pyruvamide and styrene, respectively, which were used for producing a resin; the values 213 and 104 are the molecular weights of N-(2-(methacryloyloxy) ethyl)-N-methyl-pyruvamide and styrene, respectively; the value 3 is used because three hydrogen atoms are bonded in the terminal methyl group of N-(2-(methacryloyloxy)ethyl)-N-methyl-pyruvamide; and the value 5 is used because five hydrogen atoms are bonded to the benzene ring of styrene.
(2) When the carbonyl groups are acetoacetoxy groups:

$$\text{Decomposition rate (\%)} = [1-\{(S_2/S_0)/(A_2/A_0)\}] \times 100$$

wherein $S_2/S_0$ is the ratio of the area $S_2$ of the peak (δ2.3 (broad)) attributed to acetoacetoxylethyl methacrylate to the area $S_0$ of the peak (δ6.6-7.3 (broad)) attributed to styrene, the peaks being obtained by nuclear magnetic resonance spectroscopic analysis ($^1$H-NMR analysis ($CDCl_3$)) of a powder obtained by drying a resin dispersion under vacuum.
$A_1/A_0$ is as follows.

$$A_2 = (W_2/214) \times 3$$

$$A_0 = (W_0/104) \times 5$$

wherein $W_2$ and $W_0$ are the weights of acetoacetoxylethyl methacrylate and styrene, respectively, which were used for producing a resin; the values 214 and 104 are molecular weights of acetoacetoxylethyl methacrylate and styrene, respectively; the value 3 is used because three hydrogen atoms are bonded in the terminal methyl group of acetoacetoxylethyl methacrylate; and the value 5 is used because five hydrogen atoms are bonded to the benzene ring of styrene.

Curability: Each composition was applied over a glass plate to a thickness of about 40 μm (when cured) using an applicator and allowed to stand in a thermostat room at 20°C for 1 day to obtain a coating film. The coating film was immersed in propylene glycol monomethyl ether for 24 hours and then dried at 130°C for 1 hour. The curability was then evaluated from the propylene glycol monomethyl ether insoluble content (wt.%).

a: Insoluble content (wt.%) ≥ 80
b: Insoluble content (wt.%) < 80

Viscosity stability during storage: After measuring the viscosity of each composition, the composition was stored in a thermostat room at 20°C or 30°C for 1 week under sealed conditions. Thereafter, the viscosity was measured again, and compared with that before storage. The viscosity stability during storage was evaluated using the viscosity change rate R (%) calculated by the following formula. The smaller R, the better the viscosity stability. The viscosity (Pa·s) was measured using a viscometer "Vismetron" (product of Shibaura System Co. Ltd.).

$$\text{Viscosity change rate R (\%)} = [(\text{viscosity after storage}/\text{viscosity before storage})-1] \times 100$$

(Evaluation criteria)

a: $R \leq 10$
b: $10 < R \leq 30$
c: $30 < R$

Water resistance: Each coating film was immersed in constant-temperature water at 20°C for 24 hours, and

the water resistance was evaluated from the water absorption rate (%).

a: Water absorption rate (%) ≤ 100
b: Water absorption rate (%) > 100

The abbreviations m, s and b in the $^1H$-NMR analysis mean multiplet, singlet, and broad, respectively.

Example 1

Production of α-oxoamide group- and (meth)acryloyloxy group-containing monomer compound

(1) Production of 2,2-dimethoxymethyl propionate

Methyl pyruvate (841 g, 8.25 mol), trimethyl orthoformate (1,060 g, 10 mol, 1.2 mol equivalents), methanol (1,060 g), and paratoluenesulfonic acid monohydrate (7.84 g, 0.041 mol, 0.5 mol%) were added to a 5 L flask under nitrogen atmosphere, and stirred. Slow heat generation was observed. The resulting mixture was maintained at 55 to 58°C for 10 hours. About 8 g of 28% methanol solution of sodium methoxide was then added for neutralization. The resulting mixture was concentrated using an evaporator, thereby removing excess trimethyl orthoformate and methanol, and most of the generated methyl formate. Distillation was then performed to obtain 1,110 g of methyl 2,2-dimethoxypropionate as a colorless transparent liquid (7.5 mol, yield: 91%, purity: 98%, boiling point: 80°C/40 mmHg). The above reaction can be represented by the following scheme.

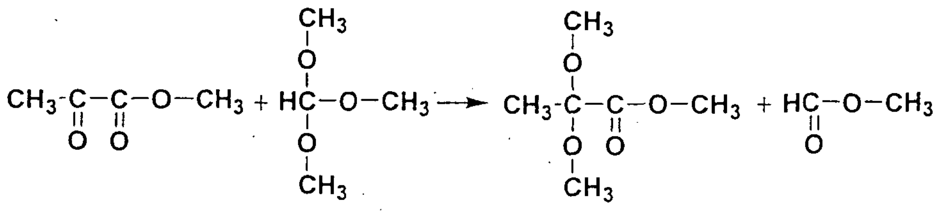

As a result of nuclear magnetic resonance spectroscopic analysis ($^1H$-NMR analysis ($CDCl_3$)) of the obtained reaction product, peaks were observed at δ: 3.82 (3H, s), 3.29 (6H, s), and 1.53 (3H, s). Further, as a result of infrared spectroscopic analysis (IR analysis), absorption was observed at ν: 3632, 2999, 2953, 2837, 1749, 1455, 1437, 1373, 1293, 1216, 1192, 1144, 1046, 976, 892, 801, 761, and 676 $cm^{-1}$.

(2) Production of N-(2-hydroxyethyl)-N-methyl-2,2-dimethoxypropionic acid amide

The methyl 2,2-dimethoxypropionate (1,110 g, 7.5 mol) obtained (1) above, and N-methyl-ethanolamine (619 g, 8.25 mol, 1.1 equivalents) were mixed in a 2 L flask under nitrogen atmosphere, and 14.5 g (0.075 mol, 1 mol%) of 28% methanol solution of sodium methoxide was further added, followed by stirring. Slow generation of heat was observed. The resulting mixture was then heated to 70°C, and maintained at 70°C for 20 hours while distilling off the generated methanol under reduced pressure. Thereafter, about 5 g of acetic acid was added for neutralization. The resulting mixture was then concentrated using an evaporator, thereby removing the generated methanol. Distillation was then performed to thereby obtain 1,146 g of N-(2-hydroxyethyl)-N-methyl-2,2-dimethoxy propionic acid amide as a pale yellow transparent liquid (6.0 mol, yield: 80%, purity: 96%, boiling point: 135°C/2 mmHg).

The above reaction can be represented by the following scheme.

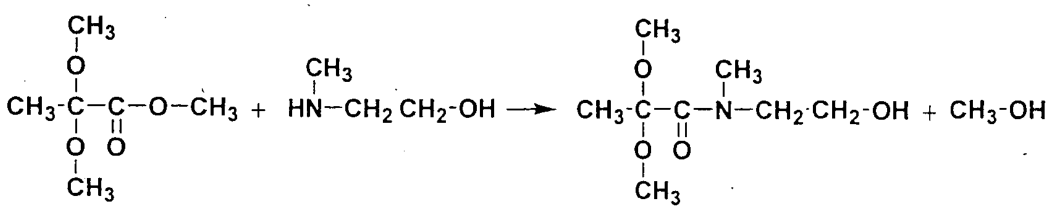

As a result of nuclear magnetic resonance spectroscopic analysis ($^1$H-NMR analysis ($CDCl_3$)) of the obtained reaction product, peaks were observed at δ: 3.58-3.84 (4H, m), 2.99-3.32 (9H, m), 2.50-2.99 (1H, b), and 1.54-1.56 (3H, m). Further, as a result of infrared spectroscopic analysis (IR analysis), absorption was observed at v: 3445, 2945, 2837, 1634, 1498, 1456, 1437, 1404, 1375, 1226, 1149, 1106, 1041, 892, 747, and 655 $cm^{-1}$.

(3) Production of N-(2-(methacryloyloxy)ethyl)-N-methyl-2,2-dimethoxypropionic acid amide

The N-(2-hydroxyethyl)-N-methyl-2,2-dimethoxypropionic acid amide obtained in (2) above (135 g, 0.71 mol), methyl methacrylate (330 g, 3.3 mol, 4.6 equivalents), an N-oxyl compound represented by Formula (VIII)

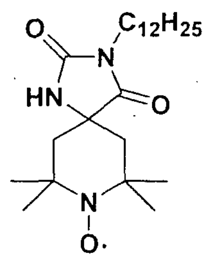

(VIII)

(0.23 g), hydroquinone monomethyl ether (0.23 g), and dioctyltin oxide (2.6 g) were added to a 500 ml flask equipped with a rectifying tower, and stirred. The resulting mixture was heated to 105°C while introducing air. Then, while maintaining the temperature at 105°C, the methanol generated with the passage of time was distilled off over 12 hours during which the pressure was gradually reduced. The pressure was finally reduced to 25 mmHg.

Subsequently, the N-oxyl compound of Formula (VIII) (0.09 g), hydroquinone monomethyl ether (0.09 g), and di-t-butyl methyl phenol (0.09 g) were added, and then distillation was performed while introducing air, to thereby obtain 172 g of N-(2-(methacryloyloxy)ethyl)-N-methyl-2,2-dimethoxypropionic acid amide as a pale yellow transparent liquid (yield: 94%, purity: 99%, boiling point:142°C/2 mmHg).

The above reaction can be represented by the following scheme.

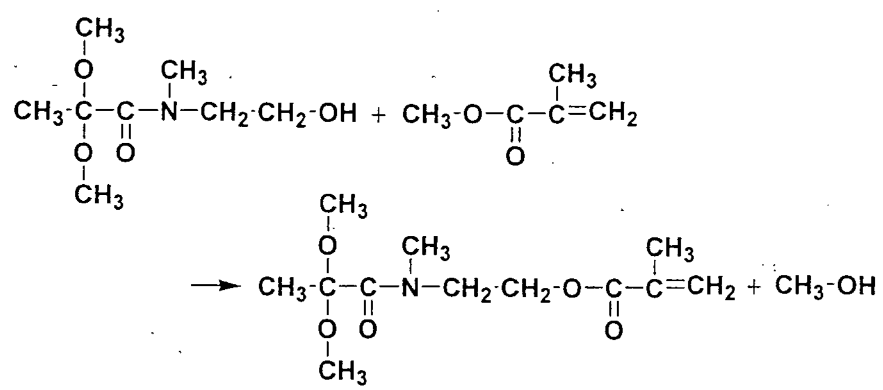

As a result of nuclear magnetic resonance spectroscopic analysis ($^1$H-NMR analysis ($CDCl_3$)) of the obtained reaction product, peaks were observed at δ: 6.11 (1H, m), 5.59 (1H, m) 4.35 (2H, m), 3.68-3.90 (2H, m), 3.28 (6H, s), 3.03-3.28 (3H, m), 1.95 (3H, m), and 1.51 (3H, m). Further, as a result of infrared spectroscopic analysis (IR analysis), absorption was observed at u: 3498, 2946, 2834, 1719, 1652, 1455, 1400, 1373, 1319, 1296, 1164, 1104, 1040, 945, 892, 815, 746, and 651 $cm^{-1}$.

(4) Production of N-(2-(methacryloyloxy)ethyl)-N-methyl-pyruvamide

To a 2 L flask were added 166 g of N-(2-(methacryloyloxy)ethyl)-N-methyl-2,2-dimethoxypropionic acid amide obtained in (3) above, and 495 g of about 4% hydrochloric acid, followed by vigorous agitation. After continuing agitation at 25°C for 5 hours, the resulting mixture was neutralized with a 10% aqueous sodium hydrogencarbonate solution to adjust the pH to about 6. After adding 400 ml.of ethyl acetate, sodium chloride was added to the aqueous phase, and

the product was extracted to the organic phase by liquid-liquid separation. Further, the cycle of adding 200 ml of ethyl acetate and extracting the product to the organic phase by liquid-liquid separation was repeated 4 times. The organic phases were then collected, and concentrated using an evaporator. Thereafter, 0.04 g of hydroquinone monomethyl ether, 0.04 g of di-t-butyl methyl phenol, and 0.14 g of N-oxyl compound as used in (3) were added, and distillation was performed while introducing air to obtain 122 g of N-(2-(methacryloyloxy)ethyl)-N-methyl-pyruvamide as a pale yellow transparent liquid (yield: 90%, purity: 96%, boiling point: 138°C/3 mmHg), which is characterized by being free from acetoacetoxy groups. The above reaction can be represented by the following scheme.

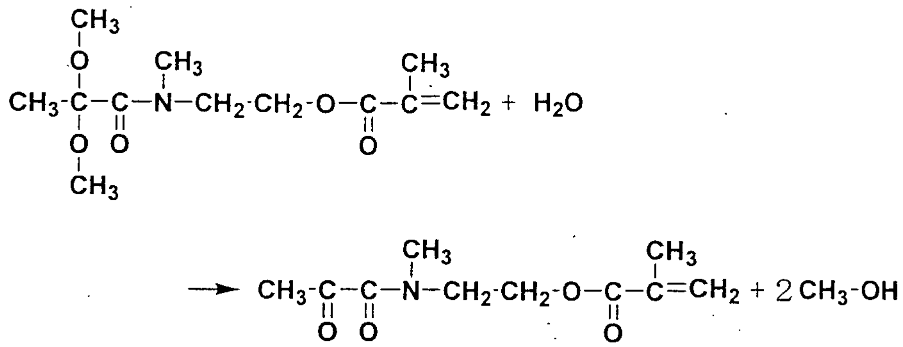

As a result of nuclear magnetic resonance spectroscopic analysis ($^1$H-NMR analysis ($CDCl_3$)) of the obtained reaction product, peaks were observed at δ: 6.11 (1H, m), 5.61 (1H, m), 4.32-4.37 (2H, m), 3.66-3.73 (2H, m), 3.05-3.09 (3H, m), 2.42 (3H, m), and 1.95 (3H, s). Further, as a result of infrared spectroscopic analysis (IR analysis), absorption was observed at ν: 3518, 2959, 1718, 1644, 1492, 1453, 1408, 1354, 1318, 1296, 1163, 1103, 1019, 947, 815, 745, and 656 $cm^{-1}$.

Example 2

Production of α-oxoamide group- and (meth)acryloyloxy group-containing monomer compound

(1) Production of N-(2-(methacryloyloxy)ethyl)-N-methyl-2,2-dimethoxypropionic acid amide

To a 300 ml flask equipped with a rectifying tower were added 57.3 g (0.3 mol) of N-(2-hydroxyethyl)-N-methyl-2,2-dimethoxypropionic acid amide obtained in Example 1 (2) above, 150 g (1.5 mol, 5 equivalents) of methyl methacrylate, 0.1 g.of N-oxyl compound of Formula (VIII) as used in Example 1, 0.1 g of hydroquinone monomethyl ether, and 1.1 g of dioctyltin oxide, followed by stirring. The resulting mixture was heated to 105°C, while introducing air. Then while maintaining the temperature at 105°C, the methanol generated with passage of time was distilled off over a period of 12 hours during which the pressure was gradually reduced. The pressure was finally reduced to 25 mmHg.

Thereafter, 0.04 g of N-oxyl compound of Formula (VIII), 0.04 g of hydroquinone monomethyl ether, and 0.04 g of di-t-butyl methyl phenol were added, and then distillation was performed while introducing air, to thereby obtain 73 g of N-(2-(methacryloyloxy)ethyl)-N-methyl-2,2-dimethoxypropionic acid amide as a pale yellow transparent liquid (yield: 94%, purity: 99%, boiling point: 142°C/2 mmHg).

The above reaction was the same as Step (3) in Example 1, and as a result of nuclear magnetic resonance spectroscopic analysis and infrared spectroscopic analysis, absorption peaks were observed at the same positions as in Example 1.

(2) Production of N-(2-(methacryloyloxy)ethyl)-N-methyl-pyruvamide

The N-(2-(methacryloyloxy)ethyl)-N-methyl-2,2-dimethoxypropionic acid amide (73 g) and about 4% hydrochloric acid (220 g) were added to a 1 L flask, followed by vigorous agitation. After continuing agitation at 25°C for 5 hours, the resulting mixture was neutralized with a 10% sodium hydrogencarbonate solution to adjust the pH to about 6. After adding 180 ml of ethyl acetate, sodium chloride was added to the aqueous phase, and the product was extracted to the organic phase by liquid-liquid separation. Further, the cycle of adding 90 ml of ethyl acetate and extracting the product to the organic phase by liquid-liquid separation was repeated 4 times. Subsequently, the organic phases were collected, and concentrated using an evaporator. Subsequently, 0.02 g of hydroquinone monomethyl ether, 0.02 g of di-t-butyl methyl phenol, and 0.06 g of N-oxyl compound of Formula (VIII) were added, and then distillation was performed

while introducing air, to thereby obtain 55 g of N-(2-(methacryloyloxy)ethyl)-N-methyl-pyruvamide as a pale yellow transparent liquid (yield: 90%, purity: 99%, boiling point: 138°C/3 mmHg).

The above reaction was the same as Step (4) in Example 1, and as a result of nuclear magnetic resonance spectroscopic analysis and infrared spectroscopic analysis of the obtained liquid, absorption peaks were observed at the same positions as in Example 1.

Example 3

Production of polymer by polymerization in organic solvent

Thirty grams of ethylene glycol monobutyl ether as a solvent was added to a 200 ml flask purged with nitrogen, and heated to 85°C with stirring. A mixture of 40 g of N-(2-(methacryloyloxy)ethyl)-N-methyl-pyruvamide obtained in Example 1 and 1.5 g of 2,2'-azobis(2,4-dimethylvaleronitrile) was added over a period of 4 hours using a syringe. Thereafter, a mixture of 30 g of ethylene glycol monobutyl ether and 0.5 g of 2,2'-azobis(2,4-dimethylvaleronitrile) was added over a period of 3 hours in the same manner. After aging for 1 hour, the resulting mixture was cooled to room temperature, thereby giving a viscous liquid.- The heating residue of the liquid was calculated as 40.3%, and the polymerization conversion of the monomer was calculated as about 98%, indicating that a polymer of N-(2-(methacryloyloxy) ethyl)-N-methyl-pyruvamide was produced. The molar concentration of α-oxoamide groups in the polymer was about 4.5 mol/kg.

Example 4

Production of polymer by polymerization in organic solvent

Polymerization was performed in the same manner as in Example 3 except that the N-(2-(methacryloyloxy) ethyl)-N-methyl-pyruvamide obtained in Example 2 was used in place of the N-(2-(methacryloyloxy)ethyl)-N-methyl-pyruvamide obtained in Example 1, thereby giving a viscous liquid. The heating residue of the liquid was calculated as 40.4%, and the polymerization conversion of the monomer was calculated as about 98%, indicating that a polymer of N-(2-(methacryloyloxy)ethyl)-N-methyl-pyruvamide was produced. The molar concentration of α-oxoamide groups in the polymer was about 4.5 mol/kg.

Example 5

Production of polymer by polymerization in organic solvent

Thirty grams of ethylene glycol monobutyl ether as a solvent was added to a 200 ml flask purged with nitrogen, and heated to 85°C with stirring. A mixture of 10 g of N-(2-(methacryloyloxy)ethyl)-N-methyl-pyruvamide obtained in Example 2, 10 g of methyl methacrylate, 2 g of 2-hydroxyethyl acrylate, 8 g of 2-ethylhexyl acrylate, 10 g of 2-ethylhexyl methacrylate, and 1.5 g of 2,2'-azobis(2,4-dimethylvaleronitrile) as a polymerization initiator was added over a period of 4 hours using a syringe. Thereafter, a mixture of 30 g of ethylene glycol monobutyl ether and 0.5 g of 2,2'-azobis(2,4-dimethylvaleronitrile) was added over a period of 3 hours in the same manner. After aging for 1 hour, the resulting mixture was cooled to room temperature, thereby giving a viscous liquid. The heating residue of the liquid was calculated as 40.4%, and the polymerization conversion of the monomers was calculated as about 98%, indicating that a polymer of N-(2-(methacryloyloxy)ethyl)-N-methyl-pyruvamide was produced. The molar concentration of α-oxoamide groups in the copolymer was about 1.1 mol/kg.

Example 6

Production of α-oxoamide group-containing resin dispersion (A-1)

Deionized water (28 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)-N-methyl-pyruvamide, 41.6 g of methyl methacrylate, 14 g of styrene, 23.5 g of butyl acrylate, 14.6 g of 2-ethylhexyl acrylate, 51.6 g of deionized water, 6.6 g of "Newcol N-707SF", and 0.3 g of sodium persulfate as a-polymerization initiator was added over a period of 4 hours using a syringe. Thereafter, a mixture of 5.2 g of deionized water and 0.1 g of ammonium persulfate was added over a period of 30 minutes in the same manner. After aging for 2 hours, the resulting mixture was cooled to room temperature, to thereby give white resin dispersion (A-1) in which the resin was emulsified and dispersed. The heating residue of the

resin dispersion was calculated as about 52%, and the polymerization conversion of the monomers was calculated as about 99%, indicating that a copolymer of N-(2-(methacryloyloxy)ethyl)-N-methyl-pyruvamide was produced. The molar concentration of α-oxoamide groups in the resin was about 0.3 mol/kg.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups of the resin dispersion after the test was less than 3%, which was equivalent to the decomposition rate before the test. That is, N-methyl-pyruvamide groups were scarcely decomposed by the acceleration test, demonstrating that the resin has excellent storage stability.

Example 7

Production of α-oxoamide group-containing resin dispersion (A-2)

Deionized water (28 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)-N-methyl-pyruvamide, 40.6 g of methyl methacrylate, 14 g of styrene, 23.5 g of butyl acrylate, 14.6 g of 2-ethylhexyl acrylate, 1 g of acrylic acid, 51.6 g of deionized water, 6.6 g of "Newcol N-707SF", and 0.3 g of sodium persulfate as a polymerization initiator was added over a period of 4 hours using a syringe. Thereafter, a mixture of 5.2 g of deionized water and 0.1 g of ammonium persulfate was added over a period of 30 minutes in the same manner. After aging for 2 hours, the resulting mixture was cooled to room temperature, to thereby give carboxy-containing white resin dispersion (A-2) in which the resin was emulsified and dispersed. The heating residue of the resin dispersion was calculated as about 52%, and the polymerization conversion of the monomers was calculated as about 99%, indicating that a copolymer of N-(2-methacryloyloxy)ethyl)-N-methyl-pyruvamide was produced. The molar concentration of α-oxoamide groups in the resin was about 0.3 mol/kg, and the molar concentration of carboxy groups in the resin was about 0.14 mol/kg.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups in the resin dispersion after the test was less than 3%, which was equivalent to the decomposition rate before the test. That is, N-methyl-pyruvamide groups were scarcely decomposed by the acceleration test, demonstrating that the resin has excellent storage stability.

Example 8

Production of α-oxoamide group-containing resin dispersion (A-3)

Deionized water (32 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)=N-methyl-pyruvamide, 39.1 g of methyl methacrylate, 14 g of styrene, 23.5 g of butyl acrylate, 14.6 g of 2-ethylhexyl acrylate, 2.5 g of acrylic acid, 51.6 g of deionized water, 6.6 g of "Newcol N-707SF", and 0.3 g of sodium persulfate as a polymerization initiator was added over a period of 4 hours using a syringe. Thereafter, a mixture of 5.2 g of deionized water and 0.1 g of ammonium persulfate was added over a period of 30 minutes in the same manner. After aging for 2 hours, the resulting mixture was cooled to room temperature, to thereby give carboxyl-containing white resin dispersion (A-3) in which the resin was emulsified and dispersed. The heating residue of the resin dispersion was calculated as about 51%, and the polymerization conversion of the monomers was calculated as about 99%, indicating that a copolymer of N-(2-(methacryloyloxy)ethyl)-N-methyl-pyruvamide was produced. In the resin, the molar concentration of α-oxoamide groups was about 0.3 mol/kg, and the molar concentration of carboxy groups was about 0.35 mol/kg.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups in the resin dispersion after the test was less than 3%, which was equivalent to the decomposition rate before the test. That is, N-methyl-pyruvamide groups were scarcely decomposed by the acceleration test, demonstrating that the resin has excellent storage stability.

Example 9

Production of α-oxoamide group-containing resin dispersion (A-4)

Triethylamine (0.03 g) was added to 70 g of resin dispersion (A-2) obtained in Example 7 to obtain a carboxylate group-containing resin dispersion (A-4). In the resin, the molar concentration of α-oxoamide groups was about 0.3 mol/kg, and the total molar concentration of carboxy and carboxylate groups was about 0.14 mol/kg.

Example 10

Production of α-oxoamide group-containin resin dispersion (A-5N)

Deionized water (32 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)-N-methyl-pyruvamide, 41.6 g of methyl methacrylate, 14 g of styrene, 23 g of butyl acrylate, 14.6 g of 2-ethylhexyl acrylate; 0.5 g of "Light Ester P-1M" (tradename of Kyoeisha Chemical, Co., Ltd.; main component: ester of 2-hydroxyethyl methacrylate with phosphoric acid; amount of acid that can be titrated with potassium hydroxide: about 8.6 mol/kg), 51.6 g of deionized water, 6.6 g of "Newcol N-707SF", and 0.3 g of sodium persulfate as a polymerization initiator was added over a period of 4 hours using a syringe. Thereafter, a mixture of 5.2 g of deionized water and 0.1g of ammonium persulfate was added over a period of 30 minutes in the same manner. After aging for 2 hours, the resulting mixture was cooled to room temperature, to thereby give phosphoric acid group-containing white resin dispersion (A-5) in which the resin was emulsified and dispersed. The heating residue of the resin dispersion was calculated as about 51%, and the polymerization conversion of the monomers was calculated as about 99%, indicating that a copolymer of N-(2-(methacryloyloxy)ethyl)-N-methyl-pyruvamide was produced.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups in the resin dispersion after the test was less than 3%, which was equivalent to the decomposition rate before the test. That is, N-methyl-pyruvamide groups were scarcely decomposed by the acceleration test, demonstrating that the resin has excellent storage stability.

Triethylamine (0.03 g) was added to 50 g of resin dispersion (A-5) to obtain N-methyl-pyruvamide group- and phosphate group-containing resin dispersion (A-5N). The molar concentration of α-oxoamide groups in the resin was about 0.3 mol/kg.

Example 11

Production of α-oxoamide group-containing resin dispersion (A-6N)

Deionized water (32 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)-N-methyl-pyruvamide, 41.6 g of methyl methacrylate, 14 g of styrene, 23 g of butyl acrylate, 14.6 g of 2-ethylhexyl acrylate, 0.5 g of 2-acrylamide-2-methylsulfonic acid, 51.6 g of deionized water, 6.6 g of "Newcol N-707SF", and 0.3 g of sodium persulfate as a polymerization initiator was added over a period of 4 hours using a syringe. Thereafter, a mixture of 5.2 g of deionized water and 0.1 g of ammonium persulfate was added over a period of 30 minutes in the same manner. After aging for 2 hours, the resulting mixture was cooled to room temperature, to thereby give sulfonic acid group-containing white resin dispersion (A-6) in which the resin was emulsified and dispersed. The heating residue of the resin dispersion was calculated as about 51%, and the polymerization conversion of the monomers was calculated as about 99%, indicating that a copolymer of N-(2-(methacryloyloxy)ethyl)-N-methyl-pyruvamide was produced.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups in the resin dispersion after the test was less than 3%, which was equivalent to the decomposition rate before the test. That is, N-methyl-pyruvamide groups were scarcely decomposed by the acceleration test, demonstrating that the resin has excellent storage stability.

Triethylamine (0.03 g) was added to 50 g of resin dispersion (A-6) to obtain N-methyl-pyruvamide group- and sulfonate group-containing resin dispersion (A-6N). The molar concentration of α-oxoamide groups in the resin was about 0.3 mol/kg.

Example 12

Production of α-oxoamide group-containing resin dispersion (A-7)

Deionized water (32 g) and "Newcol N-707SF" (tradename of Nippon Nyukazai Co., Ltd.; polyoxyethylene phenyl ether sulfuric acid ester-based emulsifier; 0.08 g) were added to a 300 ml flask purged with nitrogen, and heated to 85°C with stirring. A mixture of 6.3 g of N-(2-(methacryloyloxy)ethyl)-N-methyl-pyruvamide, 39.1 g of methyl methacrylate, 14 g of styrene, 23 g of butyl acrylate, 14.6 g of 2-ethylhexyl acrylate, 2.5 g of acrylic acid, 0.5 g of "Blenmer PME-100" (tradename of Nippon Oil & Fat Co., Ltd.; methoxy polyethylene glycol monomethacrylate), 51.6 g of deionized water, 6.6 g of "Newcol N-707SF", and 0.3 g of sodium persulfate as a polymerization initiator was added over a period of 4 hours using a syringe. Thereafter, a mixture of 5.2 g of deionized water and 0.1 g of ammonium persulfate was added over a period of 30 minutes in the same manner. After aging for 2 hours, the resulting mixture was cooled to room temperature, to thereby give carboxy group- and polyoxyethylene group-containing white resin dispersion (A-7) in which the resin was emulsified and dispersed. The heating residue of the resin dispersion was calculated as about 51%, and the polymerization conversion of the monomers was calculated as about 99%, indicating that an N-methyl-pyruvamide group- and polyoxyethylene group-containing copolymer was produced. In the resin, the molar concentration of α-oxoamide groups was about 0.3 mol/kg, and the molar concentration of carboxy groups was about 0.35 mol/kg.

Part of the resin dispersion was dried under reduced pressure, and the obtained solids were analyzed by nuclear magnetic resonance spectroscopy, and the decomposition rate of N-methyl-pyruvamide groups during polymerization in water was calculated. As a result, the decomposition rate was as low as less than 3%.

To test the storage stability of the resin dispersion obtained above, a decomposition acceleration test was conducted in which part of the resin dispersion was maintained at 80°C for 20 hours. The decomposition rate of N-methyl-pyruvamide groups in the resin dispersion after the test was less than 3%, which was equivalent to the decomposition rate before the test. That is, N-methyl-pyruvamide groups were scarcely decomposed by the acceleration test, demonstrating that the resin has excellent storage stability.

Production Example 1

Production of emulsified dispersion (B-1) of compound having primary amino groups partially neutralized with Brönsted acid

"Epikote 1001" (tradename of Japan Epoxy Resin Co., Ltd.; bisphenol A-type epoxy resin; 118.8 g), "Epikote 828EL" (tradename of Japan Epoxy Resin Co., ltd.; bisphenol A-type epoxy resin; 225 g), and methyl isobutyl ketone (297 g) were added to a 5 L flask purged with nitrogen, and heated to 70°C to give a solution. The solution was immediately cooled, and a mixture of 20 g of "Jeffamine D-400" (tradename of Mitsui Fine Chemicals, Inc.; aliphatic diamine derived from polypropylene glycol; amount of primary amine: 4.93 mol/kg; total amount of amines: 4.99 mol/kg; amount of active hydrogen: 10 mol/kg) and 58.1 g of 2-ethylhexyl amine was added over a period of 5 minutes. After heating to 80°C and maintaining that temperature for 1 hour, the resulting mixture was rapidly cooled to 40°C, and 129.2 g of diketimine obtained by dehydration of diethylenetriamine with methyl isobutyl ketone, was added. After heating to 80°C and maintaining that temperature for 1 hour, the resulting mixture was further heated to 100°C and maintained at that temperature for 2 hours. Then, after cooling to 80°C, 162 g of water and 13.5 g of acetic acid were added, and the resulting mixture was maintained at 80°C to hydrolyze the ketimine moiety into amino groups partially neutralized with acetic acid. After adding 1227 g of 80°C deionized water, about 660 g of methyl isobutyl ketone and water was azeotropically removed at 50 to 80°C under reduced pressure, and the remainder was diluted with deionized water to a heating residue of 21%, to thereby give emulsified dispersion (B-1) (heating residue: about 21%) of a compound having primary amino groups partially neutralized with Brönsted acid. The molar concentration of primary amino groups partially neutralized with Bröensted acid in the resin was about 1.7 mol/kg.

Production Example 2

Production of emulsified dispersion (B-2) of compound having primary amino groups partially neutralized with Bröensted acid

"Epikote 1001" (tradename of Japan Epoxy Resin Co., Ltd.; bisphenol A-type epoxy resin, 118.8 g), "Epikote 828EL" (tradename of Japan Epoxy Resin Co., Ltd.; bisphenol A-type epoxy resin; 225 g), and methyl isobutyl ketone (297 g) were added to a 5 L flask purged with nitrogen, and heated to 70°C to give a solution. The solution was immediately cooled, and a mixture of 20 g of "Jeffamine D-400" (tradename of Mitsui Fine Chemicals, Inc.; aliphatic diamine derived from polypropylene glycol; amount of primary amine: 4.93 mol/kg; total amount of amines: 4.99 mol/kg; amount of active hydrogen: 10 mol/kg) and 58.1 g of 2-ethylhexyl amine was added over 5 minutes. After heating to 80°C and maintaining that temperature for 1 hour, the resulting mixture was rapidly cooled to 40°C, and 122.7 g of diketimine obtained by dehydration of diethylenetriamine with methyl isobutyl ketone and 3 g of 2,2'-thiodiethanol were added. After heating to 80°C and maintaining that temperature for 1 hour, the resulting mixture was further heated to 100°C and maintained at that temperature for 2 hours. After cooling to 80°C, 162 g of water and 27 g of acetic acid were added, and the resulting mixture was maintained at 80°C to hydrolyze the ketimine moiety into amino groups partially neutralized with acetic acid. After adding 1,227 g of 80°C deionized water, about 660 g of methyl isobutyl ketone and water was azeotropically removed at 50 to 80°C under reduced pressure, and the remainder was diluted with deionized water to a heating residue of 21%, to thereby give emulsified dispersion (B-2) (heating residue: about 21%) of a compound having sulfonium groups and primary amino groups partially neutralized with Bröensted acid. The molar concentration of primary amino groups partially neutralized with Bröensted acid in the resin was about 1.7 mol/kg.

Production Example 3

Production of acetoacetoxy group-containing resin dispersion (C-1)

The procedure of Example 6 was followed except for using 6.3 g of acetoacetoxylethyl methacrylate (product of Eastman Co.) in place of 6.3 g of N-(2-(methacryloyloxy)ethyl)-N-methyl-pyruvamide, to obtain acetoacetoxy group-containing resin dispersion (C-1). The resin dispersion had a heating residue of about 51%. Part of the resin dispersion was dried under reduced pressure, and the obtained solids were analyzed by nuclear magnetic resonance spectroscopy, and the decomposition rate of acetoacetoxy groups during polymerization in water was calculated. The decomposition rate was as high as 44%.

To test the storage stability of the resin dispersion obtained above, a decomposition acceleration test was conducted in which the resin dispersion was maintained at 80°C for 20 hours. The decomposition rate of acetoacetoxy groups in the resin dispersion after the test was 59%, which was 15% higher than the decomposition rate before the test. That is, acetoacetoxy groups are further decomposed by the acceleration test, showing that the resin has poor storage stability.

Production Example 4

Production of 1,1-dimethyl-3-oxobutyl group-containing resin dispersion (D-1) (for comparison)

The procedure of Example 6 was followed except for using 6.3 g of N-(1,1-dimethyl-3-oxobutyl)acrylamide was used in place of 6.3 g of N-(2-(methacryloyloxy)ethyl)-N-methyl-pyruvamide, to obtain 1,1-dimethyl-3-oxobutyl group-containing resin dispersion (D-1). The resin dispersion had a heating residue of about 52%. The concentration of 1, 1-dimethyl-3-oxobutyl groups in the resin was about 0.38 mol/kg. Part of the resin dispersion was dried under reduced pressure, and the obtained solids were analyzed by nuclear magnetic resonance spectroscopy, and the decomposition rate of 1,1-dimethyl-3-oxobutyl groups during polymerization in water was calculated. The decomposition rate was as low as less than 3%.

To test the storage stability of the resin dispersion obtained above, a decomposition acceleration test was conducted in which the resin dispersion was maintained at 80°C for 20 hours. The decomposition rate of 1,1-dimethyl-3-oxobutyl groups in the resin dispersion after the test was less than 3%, which was equivalent to the decomposition rate before the test.

Examples 13 to 19

Preparation and evaluation of aqueous curable compositions

Aqueous curable compositions of Examples 13 to 19 were prepared by mixing and stirring the components shown in Table 1 in the proportions indicated therein. The tetramethylenediamine, hexamethylenediamine, and formic acid were added after being diluted ten-fold with deionized water.

Table 1

| Example | | 13 | 14 | 15 | 16 | 17 | 18 | 19 |
|---|---|---|---|---|---|---|---|---|
| | Resin dispersion (A-1) | 60 | | | | | | |
| | Resin dispersion (A-2) | | 60 | | | | | |
| | Resin dispersion (A-3) | | | 60 | | | | |
| Component (A) | Resin dispersion (A-4) | | | | 60 | | | |
| | Resin dispersion (A-5N) | | | | | 60 | | |
| | Resin dispersion (A-6N) | | | | | | 60 | |
| | Resin dispersion (A-7) | | | | | | | 60 |
| Component (B) | Tetramethylene-diamine | | | | | 0.58 | | |
| | Hexamethylene diamine | 0.52 | 0.52 | 0.52 | 0.58 | | 0.52 | 0.48 |
| 2,2,4-Trimethyl-1,3-pentanediolmono(2-methylpropanoate) | | 2 | 2 | 2 | 2 | 2 | 2 | 2 |
| Ratio of (A)/(B) (*1) | | 1.04 | 1.04 | 1.02 | 1.04 | 0.70 | 1.02 | 1.11 |
| Ratio of (acid groups in (A))/(crosslinkable groups in (B)) (*2) | | 0 | 0.25 | 0.60 | 0.22 | 0 | 0 | 0.66 |
| Concentration of acid groups in (A) (*3) | | 0 | 0.07 | 0.17 | 0.07 | 0 | 0 | 0.17 |

In the table, "acid groups in (A)" means carboxy and carboxylate groups.
The 2,2,4-Trimethyl-1,3-pentanediolmono(2-methylpropanoate) shown in Table 1 is a film formation auxiliary.
In Table 1, *1 to *3 indicate the following.

* 1: the.ratio of (the number of moles of α-oxoamide groups in component (A))/(the number of moles of crosslinkable groups in component (B))
* 2: the ratio of (the total number of moles of carboxy and carboxylate groups in component (A))/(the number of moles of crosslinkable groups in component (B))
* 3: the total molar concentration (mol/kg) of carboxy and carboxylate groups in component (A)

With respect to the obtained curable compositions, the curability, viscosity stability during storage, water resistance of the resulting coating films, were evaluated. Table 2 shows the results.

Table 2

| Example | | 13 | 14 | 15 | 16 | 17 | 18 | 19 |
|---|---|---|---|---|---|---|---|---|
| Curability | | a | a | a | a | a | a | a |
| Viscosity stability during storage | 20°C | a | a | a | a | a | a | a |
| | 30°C | b | a | a | a | b | b | a |
| Water resistance | | a | a | a | a | a | a | a |

Comparative Example 1

An aqueous curable composition of Comparative Example 1 was prepared by mixing and stirring 60 parts by weight of resin dispersion (D-1) obtained in Production Example 4, 0.66 parts by weight of hexamethylenediamine, and

2 parts by weight of 2,2,4-trimethyl-1,3-pentanediolmono(2-methylpropanoate). The hexamethylenediamine was added after being diluted ten-fold with deionized water.

With respect to the obtained curable composition, the curability, viscosity stability during storage, and water resistance of a coating film obtained therefrom were evaluated. As a result, the curability was evaluated as b, viscosity stability during storage (at 20°C and 30°C) as a, and water resistance of the coating film as b.

## Claims

1. A monomer compound containing an α-oxoamide group and a (meth)acryloyloxy group, the compound being represented by Formula (I)

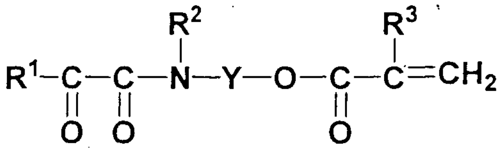 (I)

wherein Y is a $C_{1-6}$ straight-chain alkylene group which may have a $C_{1-6}$ organic group as a substituent; $R^1$ is a $C_{1-16}$ organic group; $R^2$ is a hydrogen atom or a $C_{1-8}$ organic group; and $R^3$ is a hydrogen atom or a methyl group.

2. A compound according to claim 1, wherein Y is an ethylene group which may have a $C_{1-6}$ organic group as a substituent.

3. A compound according to claim 1, wherein Y is a straight-chain propylene group which may have a $C_{1-6}$ organic group as a substituent.

4. A compound according to claim 1, wherein Y is an ethylene group, $R^1$ is a methyl group, $R^2$ is a hydrogen atom, and $R^3$ is a hydrogen atom or a methyl group.

5. A compound according to claim 1, wherein Y is an ethylene group, $R^1$ is a methyl group, $R^2$ is a $C_{1-4}$ alkyl group, and $R^3$ is a hydrogen atom or a methyl group.

6. A compound according to claim 5, wherein $R^2$ is a methyl group.

7. A process for producing a monomer compound represented by Formula (I), the process comprising the steps of:

   (1) reacting a compound represented by Formula (II)

   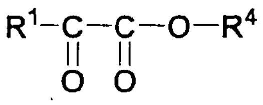 (II)

   wherein $R^1$ is a $C_{1-16}$ organic group, and $R^4$ is a hydrogen atom or a $C_{1-18}$ organic group, with at least one compound selected from the group consisting of alcohols,thiols, acetals, and orthoesters, to obtain a compound represented by Formula (III)

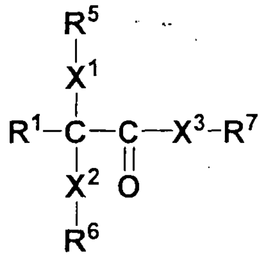

(III)

wherein $X^1$, $X^2$, and $X^3$ are each independently an oxygen atom or a sulfur atom; $R^5$ and $R^6$ are each independently a $C_{1-18}$ organic group; $R^7$ is a hydrogen atom or a $C_{1-18}$ organic group; two or three organic groups selected from $R^5$, $R^6$, and $R^7$ may be bonded to each other to form a heterocyclic ring; and $R^1$ is as defined above;
(2) reacting the compound of Formula (III) with a compound represented by Formula (IV)

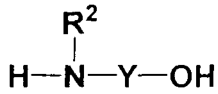

(IV)

wherein Y is a $C_{1-6}$ straight-chain alkylene group which may have a $C_{1-6}$ organic group as a substituent; and $R^2$ is a hydrogen atom or a $C_{1-8}$ organic group, to obtain a compound represented by Formula (V)

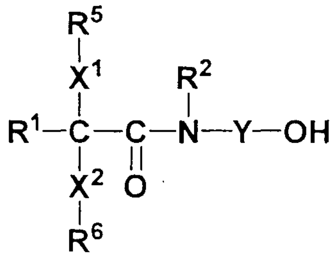

(V)

wherein $X^1$, $X^2$, Y, $R^1$, $R^2$, $R^5$, and $R^6$ are as defined above;
(3) reacting the compound of Formula (V) with a (meth)acrylic acid ester, (meth)acrylic acid, (meth)acrylic acid halide, or (meth)acrylic anhydride, to obtain a compound represented by Formula (VI)

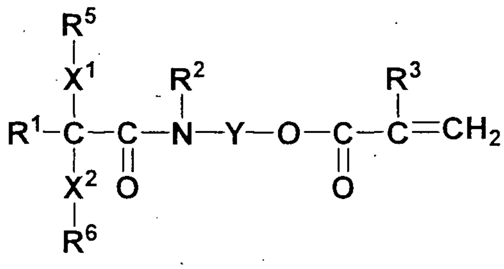

(VI)

wherein $X^1$, $X^2$, Y, $R^1$, $R^2$, $R^5$ and $R^6$ are as defined above; and $R^3$ is a hydrogen atom or a methyl group; and
(4) reacting the compound of Formula (VI) with water, in a dilute acid or in the presence of an acid compound, to obtain an α-oxoamide group- and (meth)acryloyloxy group-containing monomer compound represented by Formula (I)

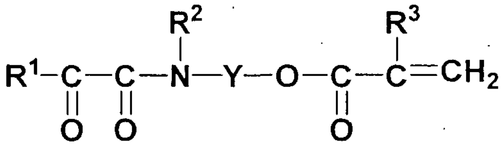

(I)

wherein Y, $R^1$, $R^2$, and $R^3$ are as defined above.

**8.** A process according to claim 7, wherein, in Step 3, the compound of Formula (V) is reacted with a (meth)acrylic acid ester in the presence of a tin catalyst, to obtain the compound of Formula (VI).

**9.** A process according to claim 8, wherein the tin catalyst is at least one organic tin compound selected from the group consisting of dialkyltin oxides, dialkyltin dicarboxylates, monoalkyltin tricarboxylates, monoalkyltin oxides, tin dicarboxylates, and organic distannoxanes.

**10.** The process according to claim 9, wherein the tin catalyst is a dialkyltin oxide.

**11.** The process according to claim 10, wherein the dialkyltin oxide is a dioctyltin oxide.

**12.** A polymer obtained by homopolymerizing the monomer compound according to claim 1 or by copolymerizing the monomer compound with a vinyl monomer or monomers other than the compound, the polymer having α-oxoamide groups represented by Formula (VII)

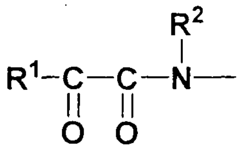

(VII)

wherein $R^1$ is a $C_{1-16}$ organic group, and $R^2$ is a hydrogen atom or a $C_{1-8}$ organic group.

**13.** The polymer according to claim 12, wherein the molar concentration of α-oxoamide groups of Formula (VII) in the polymer is 0.05 to 5.5 mol/kg.

**14.** The polymer according to claim 12, wherein the homopolymerization or copolymerization is carried out in an organic solvent.

**15.** The polymer according to claim 12, wherein the homopolymerization or copolymerization is carried out in an emulsified state in water.

**16.** The polymer according to claim 12, the polymer further containing at least one anionic group selected from the group consisting of carboxylate groups, phosphate groups, and sulfonate groups.

**17.** The polymer according to claim 12, the polymer further containing at least one nonionic hydrophilic group selected from the group consisting of polyoxyethylene groups, polyoxypropylene groups, and polyoxyethylene polyoxypropylene groups.

**18.** An aqueous curable composition comprising:

(A) the polymer according to claim 12, and
(B) a compound containing, per molecule, at least two crosslinkable groups of at least one kind selected from the group consisting of primary amino groups, secondary amino groups, and ammonium groups.

**19.** An aqueous curable composition according to claim 18, wherein the primary amino group or groups and/or secondary

amino group or groups in the compound (B) are neutralized with a Brönsted acid.

**20.** An aqueous curable composition according to claim 18, wherein the compound (B) is at least one compound selected from the group consisting of (polyamino)alkanes, (polyamino)polyether compounds, poly(ethyleneimine)s, and poly (aminoalkene)s.

**21.** An aqueous curable composition according to claim 18, wherein the compound (B) is a cationic resin produced using, as starting materials, an amine compound and a compound having at least two glycidyl groups per molecule.

**22.** An aqueous curable composition according to claim 18, wherein the compound (B) further contains at least one nonionic hydrophilic group selected from the group consisting of monoalkyl polyoxyethylene groups, monoalkyl polyoxypropylene groups, and monoalkyl polyoxyethylene polyoxypropylene groups.

**23.** An aqueous curable composition according to claim 18, wherein the molar concentration of α-oxoamide groups of Formula (VII) in the polymer (A) is 0.05 to 5.5 mol/kg; and wherein the molar concentration of crosslinkable groups in the compound (B) is 0.05 to 35 mol/kg.

**24.** An aqueous curable composition according to claim 18, wherein the polymer (A) further contains carboxy and carboxylate groups in a total molar concentration of 0.05 to 2 mol/kg.

**25.** An aqueous curable composition according to claim 18, wherein the ratio of the polymer (A) to the compound (B) is such that the ratio of α-oxoamide groups of Formula (VII) in the polymer (A) to crosslinkable groups in the compound (B) is (the number of moles of the α-oxoamide groups)/(number of moles of moles of the crosslinkable groups) = 0.3 to 3.

**26.** An aqueous curable composition according to claim 18, wherein the polymer (A) further contains carboxy and carboxylate groups; and wherein the polymer (A) and the amine compound (B) are contained in such a ratio that the ratio of the total number of moles of the carboxy and carboxylate groups to the number of moles of crosslinkable groups in the compound (B) is (the total number of moles of the carboxy and carboxylate groups)/(the number of moles of the crosslinkable groups) = 0.05 to 10.

**27.** A method for forming a coating film, the method comprising applying an aqueous curable composition according to claim 18 by electrodeposition.

**INTERNATIONAL SEARCH REPORT**

International application No.
PCT/JP2005/018043

A. CLASSIFICATION OF SUBJECT MATTER
*C07C235/80* (2006.01), *C07C231/12* (2006.01), *C08F20/36* (2006.01), *C09D133/14* (2006.01)

According to International Patent Classification (IPC) or to both national classification and IPC

B. FIELDS SEARCHED

Minimum documentation searched (classification system followed by classification symbols)
*C07C235/80* (2006.01), *C07C231/12* (2006.01), *C08F20/36* (2006.01), *C09D133/14* (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2004-75917 A (Fuji Xerox Co., Ltd.), 11 March, 2004 (11.03.04), & US 2004/0039109 A1 | 1-27 |
| A | JP 9-316046 A (Kuraray Co., Ltd.), 09 December, 1997 (09.12.97), (Family: none) | 1-27 |
| A | JP 2000-159736 A (Kohjin Co., Ltd.), 13 June, 2000 (13.06.00), (Family: none) | 1-27 |
| A | JP 2003-40927 A (Yugen Kaisha Yamaguchi TLO), 13 February, 2003 (13.02.03), (Family: none) | 1-27 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 November, 2005 (04.11.05) | Date of mailing of the international search report<br>15 November, 2005 (15.11.05) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/018043

C (Continuation). DOCUMENTS CONSIDERED TO BE RELEVANT

| Category* | Citation of document, with indication, where appropriate, of the relevant passages | Relevant to claim No. |
|---|---|---|
| A | JP 56-129225 A (Armstrong Cork Co.), 09 October, 1981 (09.10.81), (Family: none) | 1-27 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000159736 A
- US 3984299 A
- US 4017438 A
- JP 59043013 A